# EUROPEAN PATENT SPECIFICATION

(11) **EP 4 006 083 B1**
(45) Date of publication and mention of the grant of the patent: **22.05.2024**
(21) Application number: 21175596.2
(22) Date of filing: 25.05.2021
(51) Int. Cl.: A61K 8/02, A61K 8/85, A61K 8/88, A61Q 19/00, C08K 5/101, C08K 5/13, C08K 5/1545, C08J 3/12, A61K 8/73, C08K 5/11, C08L 1/14

(54) **A RESIN PARTICLE**
HARZPARTIKEL
PARTICULE DE RÉSINE

(30) Priority: 30.11.2020 JP 2020198972
(43) Date of publication of application: 01.06.2022
(73) Proprietor: Fujifilm Business Innovation Corp., Tokyo (JP)
(72) Inventor: YAO, Kenji, Tokyo (JP); YOSHIKAWA, Hideaki, Tokyo (JP); OKI, Masahiro, Tokyo (JP); YOSHIDA, Kazusei, Tokyo (JP); TAGUCHI, Tetsuya, Tokyo (JP)
(74) Representative: Haseltine Lake Kempner LLP

(56) References cited:
- EP-A1- 1 582 194
- EP-A1- 1 939 242
- EP-A1- 3 613 794
- US-A1- 2014 018 530
- KIUCHI YUKIHIRO ET AL: "Improvement in impact strength of modified cardanol-bonded cellulose thermoplastic resin by using olefin resins", JOURNAL OF APPLIED POLYMER SCIENCE, vol. 131, no. 3, 5 February 2014 (2014-02-05), XP055856979, US ISSN: 0021-8995, DOI: 10.1002/app.39829 Retrieved from the Internet: URL:https://onlinelibrary.wiley.com/doi/fu ll-xml/10.1002/app.39829>
- Toray Industries: "Toray's nylon particle", , 2019, XP002805685, Retrieved from the Internet: URL:https://www.toray.jp/plastics/en/parti culate/index.html [retrieved on 2022-02-15]

## Description

### BACKGROUND

### 1. Technical Field

The present invention relates to a resin particle.

### 2. Related Art

JP2007-231050A discloses a resin composition containing a hydroxy acid-modified cellulose acylate and a thermoplastic resin different from the hydroxy acid-modified cellulose acylate. The hydroxy acid-modified cellulose acylate is composed of a cellulose acylate and a graft chain formed by graft-polymerizing a hydroxy acid component on the hydroxy group of the cellulose acylate. In the hydroxy acid-modified cellulose acylate, the ratio of the graft-polymerized hydroxy acid component is 0.1 mol to 5 mol on average in terms of hydroxy acid with respect to 1 mol of glucose units constituting the cellulose acylate.

JP6187653B1 discloses a cellulose acylate which has a volume average particle diameter D50 of 72 µm or more and 100 µm or less, a degree of polymerization of 131 or more and 350 or less, and a degree of substitution of 2.1 or more and 2.6 or less, in which the volume average particle diameter D50 is measured by using a laser diffraction type particle size distribution measurement device.

EP 3613794 A1 relates to cellulose acetate particles possessing a specified average particle size, sphericity, surface smoothness, and a total degree of acetyl substitution.

US 2014/018530 A1 relates to a cellulose resin produced by binding a hydrogenated cardanol and cellulose or a derivative thereof.

Yukihiro et al, Journal of Applied Polymer Science, vol. 131, no 3, 5 February 2014 relates to improving the impact strength of cardanol-bonded cellulose thermoplastic resin using olefin resins.

Toray Industries: "Toray's nylon particle", 2019, XP002805685 relates to spherical Nylon 12 fine particles and porous Nylon 6 particles.

EP 1582194 A1 relates to a cosmetic composition using porous polyamide particles.

EP 1939242 A1 relates to a cosmetic composition containing polyamide porous spherical particles.

### SUMMARY

An object of the present invention is to provide a resin particle excellent in heat resistance and flexibility, as compared with (i) a resin particle having a mass loss rate of 10% or more after being immersed in distilled water at 70°C for 14 days, (ii) a resin particle having a mass loss rate of less than 90% after being left in a compost at 55°C for 14 days, the compost having 1.3 mass% of nitrogen, 0.5 mass% of phosphoric acid, and 2.9 mass% of potassium with respect to a total amount of the compost, and 25 of a carbon-nitrogen ratio (C/N ratio), (iii) a resin particle having a melting point of lower than 170°C, (iv) a resin particle having a Vickers hardness of more than 900 HV, (v) a resin particle containing at least one selected from the group consisting of a cellulose acylate (A), a polyamide compound (B) and an aliphatic polyester compound (C), or (vi) a resin particle containing at least one selected from the group consisting of a cellulose acylate (A), a polyamide compound (B) and an aliphatic polyester compound (C), and an ester compound (E) in an amount of 30 mass% or more with respect to the total amount of the resin particles.

The above problems are solved by the following aspects.

The present invention is defined in and by the appended claims.

According to a first aspect of the present invention, there is provided a resin particle, in which a mass loss rate after being immersed in distilled water at 70°C for 14 days is less than 10%, and in which a mass loss rate after being left in a compost at 55°C for 14 days is 90% or more, the compost having nitrogen of 1.3 mass%, phosphoric acid of 0.5 mass%, and potassium of 2.9 mass% with respect to a total amount of the compost, and the compost having a carbon-nitrogen ratio (C/N ratio) of 25, comprising
a polyamide compound (B1) having a constituent unit having 8 or more and 15 or less carbon atoms in an amount of 50 mass% or more with respect to a total amount of the resin particle; and
an aromatic compound (D) in an amount of 3 mass% or more and less than 50 mass% with respect to the total amount of the resin particle,
wherein the aromatic compound (D) has a long-chain aliphatic group having 8 or more and 20 or less carbon atoms and at least one of a phenolic hydroxy group or a monoglycidyl ether group that directly bonds to an aromatic group of the aromatic compound (D).

Accordingly, it is possible to provide a resin particle excellent in heat resistance and flexibility, as compared with a resin particle having a mass loss rate of 10% or more after being immersed in distilled water at 70°C for 14 days, or having a mass loss rate of less than 90% after being left in a compost having the composition shown above at 55°C for 14 days.

Accordingly, it is possible to provide a resin particle excellent in heat resistance, as compared with a resin particle containing less than 50 mass% of the polyamide compound (B) having a monomer component having 8 or more and 15 or less carbon atoms with respect to the total amount of the resin particles.

Accordingly, it is possible to provide a resin particle excellent in heat resistance and flexibility, as compared with a resin particle containing the aromatic compound (D) containing a phenolic hydroxy group and a hydrocarbon group having 8 or more and 20 or less carbon atoms in an amount of less than 15 mass% or 50 mass% or more with respect to the total amount of the resin particles.

In the resin particle, the aromatic compound (D) may be a cardanol compound (D1).

Accordingly, it is possible to provide a resin particle excellent in heat resistance and flexibility, as compared with a case where the aromatic compound (D) is a phenol novolac type epoxy resin.

The resin particle may contain an ester compound (E1) having a molecular weight of 200 or more and less than 500 in an amount of 5 mass% or more and less than 30 mass% with respect to the total amount of the resin particles.

Accordingly, it is possible to provide a resin particle excellent in heat resistance, as compared with a case where the ester compound (E1) having a molecular weight of 200 or more and less than 500 is contained in an amount of less than 5 mass% or 30 mass% or more based on the total amount of the resin particles.

The ester compound (E1) may be at least one of an adipate ester or a citrate ester.

Accordingly, it is possible to provide a resin particle excellent in heat resistance and flexibility, as compared with a case where the ester compound (E1) is isopropyl sebacate.

The resin particle may contain at least one selected from the group consisting of dibutylhydroxytoluene, butylhydroxyanisole, propyl gallate, tocopherol, ascorbic acid, catechin chloroformate, a hindered phenol compound, and a phosphate ester compound.

Accordingly, it is possible to provide a resin particle excellent in heat resistance and flexibility, as compared with a case of containing pentaerythritol tetrakis(3-(3,5-di-tert-butyl-4-hydroxyphenyl)propionate), which is a compound other than dibutylhydroxytoluene, butylhydroxyanisole, propyl gallate, tocopherol, ascorbic acid, catechin chloroformate, a hindered phenol compound, and a phosphate ester compound.

The resin particle may have a first layer and a second layer provided in this order at a surface of a base particle so that the first layer is provided between the base particle and the second layer, in which the first layer contains at least one of a polyalkyleneimine, a polyallylamine, or a polyvinylamine as a cationic resin, and in which the second layer contains an anionic or nonionic hydrophobic compound.

Accordingly, it is possible to provide a resin particle excellent in heat resistance, as compared with a case where a resin particles containing the cellulose acylate (A) and an ester compound (E) having a molecular weight of 200 or more and less than 500 or resin particles containing the ester compound (E) and an oxidative biodegradable resin does not include a first layer containing at least one of a polyalkyleneimine, a polyallylamine, or a polyvinylamine as a cationic resin on the surface thereof, and a second layer containing an anionic or nonionic hydrophobic compound.

There is described herein but not claimed a resin particle having a melting point of 170°C or higher and a Vickers hardness of 900 HV or less.

Accordingly, it is possible to provide a resin particle excellent in heat resistance and flexibility, as compared with a resin particle having a melting point of lower than 170°C or a Vickers hardness of more than 900 HV

There is described herein but not claimed a resin particle containing at least one selected from the group consisting of a cellulose acylate (A), a polyamide compound (B) and an aliphatic polyester compound (C); and at least one selected from the group consisting of an aromatic compound (D) and an ester compound (E), the aromatic compound (D) containing a phenolic hydroxy group and a hydrocarbon group having 8 or more and 20 or less carbon atoms, in which the ester compound (E) is contained in an amount of 0 mass% or more and less than 30 mass% with respect to a total amount of the resin particles.

Accordingly, it is possible to provide a resin particle excellent in heat resistance and flexibility, as compared with a resin particle containing at least one selected from the group consisting of a cellulose acylate (A), a polyamide compound (B) and an aliphatic polyester compound (C), or as compared with a case where in a resin particle containing at least one selected from the group consisting of a cellulose acylate (A), a polyamide compound (B) and an aliphatic polyester compound (C), and an ester compound (E) in an amount of 30 mass% or more with respect to the total amount of the resin particles.

The resin particle according to the first aspect of the present invention may have a volume average particle diameter of 3 µm or more and 100 µm or less, and a large-diameter side particle size distribution index GSDv of 1.50 or less.

Accordingly, it is possible to provide a resin particle excellent in heat resistance and flexibility, as compared with a case where the volume average particle diameter is less than 3 µm or more than 100 µm, or the large-diameter side particle size distribution index GSDv is more than 1.50.

According to a second aspect of the present invention there is provided use of the resin particle according to the first aspect of the present invention as a cosmetic base material.

Accordingly, it is possible to provide a resin particle suitable for a cosmetic base material.

### DETAILED DESCRIPTION

Hereinafter, an exemplary embodiment as an example of the present invention will be described. These descriptions and Examples illustrate the exemplary embodiment, and do not limit the scope of the exemplary embodiment.

In the numerical ranges described stepwise in this description, an upper limit or a lower limit described in one numerical range may be replaced with an upper limit or a lower limit of another numerical range described stepwise. Further, in the numerical ranges described in the present description, the upper limit or the lower limit of the numerical range may be replaced with values shown in Examples.

In the present description, the term "step" indicates not only an independent step, and even when a step cannot be clearly distinguished from other steps, this step is included in the term "step" as long as the intended purpose of the step is achieved.

Each component may contain plural corresponding substances.

In a case of referring to the amount of each component, when there are plural substances corresponding to each component, unless otherwise specified, it refers to the total amount of the plural substances.

The term "(meth)acryl" means at least one of acryl or methacryl, and the term "(meth)acrylate" means at least one of acrylate or methacrylate.

### <A Resin Particle>

### (First Exemplary Embodiment)

A Resin particle according to the first exemplary embodiment has a mass loss rate after being immersed in distilled water at 70°C for 14 days of less than 10%, and a mass loss rate after being left in a compost having a composition shown below at 55°C for 14 days is 90% or more.

### (Composition of compost)

Nitrogen is 1.3 mass%, phosphoric acid is 0.5 mass%, and potassium is 2.9 mass% with respect to a total amount of the compost. A carbon-nitrogen ratio (C/N ratio) is 25.

With the above configuration, the resin particles according to the first exemplary embodiment are excellent in heat resistance and flexibility. The reasons are presumed as follows.

The resin particles are used in various fields because of having a high degree of freedom in molecular design and being capable of being imparted with various properties. From the viewpoints of improving durability under high temperature conditions and maintaining the shape of the particles even when the resin particles are kneaded into a resin that is a base material by kneading, it may be desired that the heat resistance is high among the properties of the resin particles. Further, from the viewpoint of preventing damage to the resin particles, it may be desired that the flexibility is high among the properties of the resin particles.

However, it has been difficult to obtain resin particles excellent in both heat resistance and flexibility. This is because it is preferable to shorten the distance between the molecules constituting the resin particles in order to increase the heat resistance, and conversely, it is preferable to increase the distance between the molecules in order to increase the flexibility.

With the above configuration, the resin particles according to the first exemplary embodiment are considered to be in the following states (1) to (4). Thereby, the resin particles according to the first exemplary embodiment are excellent in heat resistance and flexibility.
(1) A state where the molecular chains of the resin contained in the resin particles have a spiral structure and the distance between the molecules is short.

In this case, since the distance between the resin molecules is short, the mass loss rate after being immersed in the distilled water at 70°C for 14 days is less than 10%. On the other hand, gaps are likely to occur between the spiral structures, and bacteria may enter the gaps. Therefore, the mass loss rate after being left in the compost having the composition shown above at 55°C for 14 days is 90% or more.

(2) A state where the distance between the resin molecules contained in the resin particles is long, but the molecules are firmly bonded to each other.

In this case, since the resin molecules are firmly bound to each other, the movement of the molecules is prevented. Therefore, the mass loss rate after being immersed in the distilled water at 70°C for 14 days is less than 10%. Then, since the distance between the resin molecules is long, bacteria may easily enter the gaps between the molecules, and the mass loss rate after being left in the compost having the composition shown above at 55°C for 14 days is 90% or more.

(3) A state where the molecular chains of the resin contained in the resin particles are intricately intertwined.

In this case, even in the case of being hydrolyzed, the resin molecules tend to stay in the resin particles since the molecules are intertwined. Therefore, the mass loss rate after being immersed in the distilled water at 70°C for 14 days is less than 10%. Further, when the molecular chains of the resin are intricately intertwined, the surface of the resin particles tends to form irregularities, so that the surface of the resin particles tends to have many gaps. Therefore, biodegradation is likely to occur from the surface of the resin particles, and the mass loss rate after being left in the compost having the composition shown above at 55°C for 14 days is 90% or more.

(4) A state where water does not easily penetrate from the surface of the resin particles, but bacteria may enter the resin particles.

In this case, the fact that water does not easily permeate from the surface of the resin particles may mean that the distance between the molecules of the resin constituting the surface of the resin particles is short. Therefore, the mass loss rate after being immersed in the distilled water at 70°C for 14 days is less than 10%. In addition, because of being more active and smaller in size than water molecules, bacteria may enter the resin particles. Therefore, biodegradation is likely to occur from the surface of the resin particles, and the mass loss rate after being left in the compost having the composition shown above at 55°C for 14 days is 90% or more.

Since the resin particles according to the first exemplary embodiment are in the states (1) to (4) above, it is presumed that they are excellent in heat resistance and flexibility.

In the case of (1) above, the heat resistance is improved since the distance between the resin molecules is short. Further, since the gap is likely to be generated between the spiral structures, the higher-order structure is bent and the flexibility is excellent.

In the case of (2) above, since the resin molecules are firmly bound to each other, the movement of the molecules is prevented. Therefore, even when heat is applied, the movement of the molecules is prevented, so that the heat resistance is improved. Further, since the distance between the resin molecules is long, the flexibility is also excellent.

In the case of (3) above, the movement of the molecules is prevented by the intertwinement of the resin molecules. Therefore, even when heat is applied, the movement of the molecules is prevented, so that the heat resistance is improved. Further, since the resin molecules form a random coil, there may be many gaps. Therefore, the flexibility is also excellent.

In the case of (4) above, the heat resistance is improved since the distance between the resin molecules constituting the surface of the resin particles is short. Then, by making the inside of the resin particles a flexible structure, the resin particles are also excellent in flexibility.

From the above, it is presumed that, with the above configuration, the resin particles according to the first exemplary embodiment are excellent in heat resistance and flexibility.

### (Second Embodiment, not claimed)

A Resin particle according to the second embodiment has a melting point of 170°C or higher and a Vickers hardness of 900 HV or less. For example, this resin particle is obtained by the above first exemplary embodiment and the following third exemplary embodiment.

With the above configuration, the resin particles according to the second embodiment are excellent in heat resistance and flexibility. The reasons are presumed as follows.

When the melting point of the resin particle to 170°C or higher, the resin particle is difficult to melt even when heat is applied. Therefore, the heat resistance is excellent. Further, when the Vickers hardness is 900 HV or less, the flexibility is also excellent.

From the above, it is presumed that, with the above configuration, the resin particles according to the second embodiment are excellent in heat resistance and flexibility.

### (Third Exemplary Embodiment: a cellulose acylate (A) nor an aliphatic polyester compound (C) are explicitly mentioned in the appended claims.)

A Resin particle according to the third exemplary embodiment contains at least one selected from the group consisting of a cellulose acylate (A), a polyamide compound (B) and an aliphatic polyester compound (C), and at least one selected from the group consisting of an aromatic compound (D) and an ester compound (E) containing a phenolic hydroxy group and a hydrocarbon group having 8 or more and 20 or less carbon atoms.

The ester compound (E) is contained in an amount of 0 mass% or more and less than 30 mass% with respect to a total amount of the resin particles.

With the above configuration, the resin particles according to the third exemplary embodiment are excellent in heat resistance and flexibility. The reasons are presumed as follows.

When the resin particles have the above composition, the resin particles are likely to be in the states (1) to (4).

From the above, it is presumed that, with the above configuration, the resin particles according to the third exemplary embodiment are excellent in heat resistance and flexibility.

Hereinafter, resin particles corresponding to any of the resin particles according to the first, second and third exemplary embodiments (hereinafter, also referred to as "resin particles according to the present exemplary embodiment") will be described in detail. However, an example of the resin particles of the present invention may be resin particles corresponding to at least one of the first, second or third exemplary embodiments.

### (Mass Loss Rate in Distilled Water)

The resin particle according to the present exemplary embodiment has a mass loss rate of less than 10% after being immersed in distilled water at 70°C for 14 days (hereinafter, also referred to as "mass loss rate in distilled water").

The mass loss rate in distilled water is measured as follows.

5 g of particles to be measured are sealed in a bag processed with a nylon net (nylon mesh #508/585-1 µm, manufactured by AS ONE Corporation) having an opening of 1 µm. Subsequently, the mass of the bag containing the particles is measured (the obtained value is defined as WA). The bag containing the particles is placed in distilled water at 70°C and maintained at 70°C for 14 days. The bag containing the particles is taken out and left in a dry oven set to 70°C for 300 minutes for drying. After drying, the mass of the bag containing the particles is measured (the obtained value is defined as WB). The mass difference (WA-WB) in the bag containing the particles measured before and after leaving in distilled water is calculated, and the amount of mass loss of the resin particles is calculated. When the mass (that is, 5 g) of the particles sealed in the bag is 100, the ratio of the mass loss amount is calculated and used as the mass loss rate (%).

The mass loss rate in distilled water is preferably 0% or more and 7% or less, more preferably 0% or more and 5% or less, and still more preferably 0% or more and 3% or less, from the viewpoint of improving the heat resistance.

The lower limit of the mass loss rate in distilled water is identically 0%, but for example may be 0.5%. As a result of energy saving in the production process from the viewpoint of environmental impact or cost, the possibility of forming fine powder of 1 µm or less increases. Since the fine powder may pass through the net and be counted in the mass loss rate, there is a case where the lower limit of the mass loss rate in distilled water is not be 0%.

### (Mass Loss Rate in Compost)

The resin particle according to the present exemplary embodiment has a mass loss rate of 90% or more after being left in a compost having a composition shown below at 55°C for 14 days (hereinafter, also referred to as "mass loss rate in compost").

The mass loss rate in compost is measured as follows.

5 g of particles to be measured are sealed in a bag processed with a nylon net (nylon mesh #508/585-1 µm, manufactured by AS ONE Corporation) having an opening of 1 µm. Subsequently, the mass of the bag containing the particles is measured (the obtained value is defined as CA). The bag containing the particles is embedded in a compost having a composition shown below (for example, a compost bacteria bed manufactured by IRIS OHYAMAInc.) and left in a dry oven set to 55°C for 14 days. The bag containing the particles is taken out, placed in distilled water and ultrasonically washed for 1 minute, and then the mass of the bag containing the particles is measured (the obtained value is defined as CB). The mass difference (CA-CB) in the bag containing the particles measured before and after leaving in compost is calculated, and the amount of mass loss of the resin particles is calculated. When the mass (that is, 5 g) of the particles sealed in the bag is 100, the ratio of the mass loss amount is calculated and used as the mass loss rate (%).

### (Composition of compost)

Nitrogen is 1.3 mass%, phosphoric acid is 0.5 mass%, and potassium is 2.9 mass% with respect to a total amount of the compost. A carbon-nitrogen ratio (C/N ratio) is 25.

The mass loss rate in compost is preferably 91% or more and 100% or less, more preferably 93% or more and 99% or less, and still more preferably 94% or more and 98% or less, from the viewpoint of improving the flexibility.

### (Composition)

The resin particles according to the present exemplary embodiment contain a resin component and preferably further contain a plasticizer.

Examples of the resin component include a biodegradable resin.

The biodegradable resin is a resin that is decomposed into water and carbon dioxide by microorganisms. Specifically, the biodegradable resin means a resin having an aerobic condition biodegradation rate of 50% or more after one month as measured by a method according to ISO-14855-2 (2018).

The biodegradable resin is preferably at least one selected from the group consisting of a cellulose acylate (A), a polyamide compound (B) and an aliphatic polyester compound (C). Further, the biodegradable resin may contain an oxidative biodegradable resin.

In a case where at least two selected from the group consisting of the cellulose acylate (A), the polyamide compound (B) and the aliphatic polyester compound (C) are contained, the total is preferably 50 mass% or more and 96 mass% or less with respect to the total amount of the resin particles.

The plasticizer is preferably at least one selected from the group consisting of an aromatic compound (D) containing a phenolic hydroxy group and a hydrocarbon group having 8 or more and 20 or less carbon atoms and an ester compound (E) having a molecular weight of 200 or more and less than 500.

Hereinafter, the composition will be described.

### -Cellulose Acylate (A)-

The cellulose acylate (A) is a cellulose derivative in which at least a part of hydroxy groups in cellulose is substituted (acylated) by an acyl group. The acyl group is a group having a structure of -CO-R^{AC} (R^{AC} represents a hydrogen atom or a hydrocarbon group).

The resin particle according to the present exemplary embodiment preferably contains the cellulose acylate (A) in an amount of 50 mass% or more with respect to the total amount of the resin particles.

Since the cellulose acylate (A) has a cellulose skeleton, it is presumed that when the cellulose acylate (A) is contained in an amount of 50 mass% or more with respect to the total amount of the resin particles, it is easier to form the above-mentioned "(1) state where the molecular chains of the resin contained in the resin particles have a spiral structure and the distance between the molecules is short". Therefore, the resin particles are more excellent in heat resistance and flexibility.

From the viewpoint of further improving the heat resistance and the flexibility of the resin particles, the cellulose acylate (A) is preferably contained in an amount of 55 mass% or more and 95 mass% or less, more preferably contained in an amount of 65 mass% or more and 93 mass% or less, and still preferably contained in an amount of 75 mass% or more and 91 mass% or less, with respect to the total amount of the resin particles.

The cellulose acylate (A) is preferably a cellulose acylate having two or more kinds of acyl groups.

It is presumed that having two different kinds of substituents prevents the packing of cellulose acylate (A) molecules and makes it easier for gaps to be formed between the spiral structures. Therefore, by using a cellulose acylate having two or more kinds of acyl groups as the cellulose acylate (A), the flexibility of the resin particles is more excellent.

The cellulose acylate (A) is, for example, a cellulose derivative represented by the following general formula (CA).

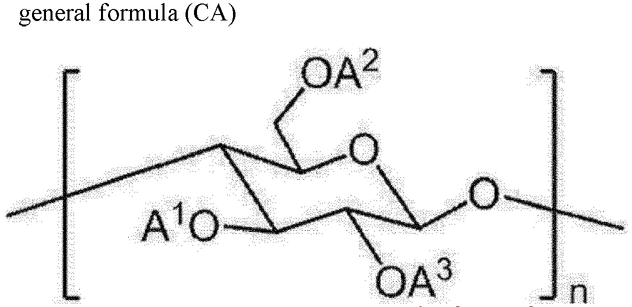

In the general formula (CA), A¹, A² and A³ independently represent a hydrogen atom or an acyl group, and n represents an integer of 2 or more. However, at least a part of n A¹s, n A²s and n A³s represents an acyl group. The n A¹s in the molecule may be all the same as, partially the same as, or different from each other. Similarly, the n A²s or the n A³s in the molecule may be all the same as, partially the same as, or different from each other.

As for the acyl group represented by A¹, A² and A³, the hydrocarbon group in the acyl group may be linear, branched or cyclic, and is preferably linear or branched, and more preferably linear.

As the acyl group represented by A¹, A² and A³, the hydrocarbon group in the acyl group may be a saturated hydrocarbon group or an unsaturated hydrocarbon group, and is more preferably a saturated hydrocarbon group.

The acyl group represented by A¹, A² and A³ is preferably an acyl group having 1 or more and 6 or less carbon atoms. That is, the cellulose acylate (A) is preferably a cellulose acylate (A) having an acyl group having 1 or more and 6 or less carbon atoms.

The acyl group represented by A¹, A² and A³ may be a group in which a hydrogen atom in the acyl group is substituted with a halogen atom (for example, a fluorine atom, a bromine atom, and an iodine atom), an oxygen atom, a nitrogen atom or the like, but is preferably unsubstituted.

Examples of the acyl group represented by A¹, A² and A³ include a formyl group, an acetyl group, a propionyl group, a butyryl group (butanoyl group), a propenoyl group, and a hexanoyl group. Among these, the acyl group is preferably an acetyl group, a propionyl group, or a butyryl group (butanoyl group) from the viewpoint of the flexibility of the resin particles.

Examples of the cellulose acylate (A) include cellulose acetate (cellulose monoacetate, cellulose diacetate (DAC), cellulose triacetate), cellulose acetate propionate (CAP), and cellulose acetate butyrate (CAB).

The cellulose acylate (A) is preferably cellulose acetate propionate (CAP) and cellulose acetate butyrate (CAB), and more preferably cellulose acetate butyrate (CAB), from the viewpoint of improving the flexibility of the resin particles.

The cellulose acylate (A) may be used alone or in combination of two or more thereof.

The weight-average degree of polymerization of the cellulose acylate (A) is preferably 200 or more and 1000 or less, more preferably 500 or more and 1000 or less, and still more preferably 600 or more and 1000 or less, from the viewpoint of improving the heat resistance and the flexibility of resin particles.

The weight-average degree of polymerization of the cellulose acylate (A) is determined from the weight average molecular weight (Mw) by the following procedure.

First, the weight average molecular weight (Mw) of the cellulose acylate (A) is measured in terms of polystyrene by gel permeation chromatography (GPC apparatus: HLC-8320GPC manufactured by Tosoh Corporation, column: TSKgelα-M) using tetrahydrofuran.

Next, the degree of polymerization of the cellulose acylate (A) is obtained by dividing by the molecular weight of the constituent unit of the cellulose acylate (A). For example, in a case where the substituent of the cellulose acylate is an acetyl group, the molecular weight of the constituent unit is 263 when the degree of substitution is 2.4, and 284 when the degree of substitution is 2.9.

From the viewpoint of improving the moldability of the resin composition and the biodegradation rate of the resin molded product, the degree of substitution of the cellulose acylate (A) is preferably 2.1 or more and 2.9 or less, more preferably 2.2 or more and 2.9 or less, still more preferably 2.3 or more and 2.9 or less, and particularly preferably 2.6 or more and 2.9 or less.

In the cellulose acetate propionate (CAP), the ratio of the degree of substitution of the acetyl group to the propionyl group (acetyl group/propionyl group) is preferably 0.01 or more and 1 or less, and more preferably 0.05 or more and 0.1 or less, from the viewpoint of improving the moldability of the resin composition and the biodegradation rate of the resin molded product.

In the cellulose acetate butyrate (CAB), the ratio of the degree of substitution of the acetyl group to the butyryl group (acetyl group/butyryl group) is preferably 0.05 or more and 3.5 or less, and more preferably 0.5 or more and 3.0 or less, from the viewpoint of improving the moldability of the resin composition and the biodegradation rate of the resin molded product.

The degree of substitution of the cellulose acylate (A) is an index indicating the degree to which the hydroxy group of cellulose is substitued by an acyl group. That is, the degree of substitution is an index indicating the degree of acylation of the cellulose acylate (A). Specifically, the degree of substitution means the intramolecular average of the number of substitutions that three hydroxy groups in the D-glucopyranose unit of the cellulose acylate are substituted with acyl groups. The degree of substitution is determined by ¹H-NMR (JMN-ECA/JEOL manufactured by RESONANCE) from the integral ratio of the peaks of cellulose-derived hydrogen and acyl group-derived hydrogen.

### -Polyamide Compound (B)-

Examples of the polyamide compound (B) include a polyamide obtained by polycondensing a dicarboxylic acid and a diamine, a polyamide obtained by condensing lactam, and a polyamide obtained by condensing ω-aminocarboxylic acid.

Examples of the dicarboxylic acid include an aliphatic dicarboxylic acid, an alicyclic dicarboxylic acid, and an aromatic dicarboxylic acid.

The number of carbon atoms of the dicarboxylic acid is preferably 2 or more and 18 or less, more preferably 5 or more and 16 or less, and still more preferably 8 or more and 15 or less.

Examples of the aliphatic dicarboxylic acid include oxalic acid, malonic acid, succinic acid, glutaric acid, adipic acid, pimelic acid, pimelic acid, azelaic acid, sebacic acid, 1,9-nonanedicarboxylic acid, 1,10-decanedicarboxylic acid, 1,11-undecanedicarboxylic acid, 1,12-dodecanedicarboxylic acid, and 1,13-tridecanedicarboxylic acid.

Examples of the alicyclic dicarboxylic acid include 1,4-cyclohexanedicarboxylic acid, 1,3-cyclohexanedicarboxylic acid, and 1,3-cyclopentanedicarboxylic acid.

Examples of the aromatic dicarboxylic acid include terephthalic acid, isophthalic acid, and naphthalenedicarboxylic acid.

Examples of the diamine include an aliphatic diamine, an alicyclic diamine, and an aromatic diamine.

The number of carbon atoms of the diamine is preferably 2 or more and 18 or less, more preferably 5 or more and 16 or less, and still more preferably 8 or more and 15 or less.

Examples of the aliphatic diamine include ethylenediamine, 1,3-propanediamine, 1,4-butanediamine, 1,5-pentanediamine, 1,6-hexanediamine, 1,7-heptanediamine, 1,8-octanediamine, 1,9-nonandiamine, 1,10-decanediamine, 1,11-undecanediamine, 1,12-dodecanediamine, 1,13-tridecanediamine, 1,14-tetradecanediamine, and 1,15-pentadecanediamine.

Examples of the alicyclic diamine include 1,4-cyclohexanediamine, 1,3-cyclohexanediamine, and 1,3-cyclopentanediamine.

Examples of the aromatic diamine include metaxylylenediamine and paraxylylenediamine.

The number of carbon atoms of the lactam is preferably 2 or more and 18 or less, more preferably 5 or more and 16 or less, and still more preferably 8 or more and 15 or less.

Examples of the lactam include ε-caprolactam, undecane lactam, and lauryl lactam.

The number of carbon atoms of the ω-aminocarboxylic acid is preferably 2 or more and 18 or less, more preferably 5 or more and 16 or less, and still more preferably 8 or more and 15 or less.

Examples of the ω-aminocarboxylic acid include 11-aminoundecanoic acid and 12-aminododecanoic acid

Specifically, the polyamide compound (B) may be at least one of an aromatic polyamide or an aliphatic polyamide.

Examples of the aromatic polyamide include MXD6 (a condensed polymer of adipic acid and m-xylylenediamine), nylon 6T (a condensed polymer of terephthalic acid and hexamethylenediamine), and nylon 9T (a polycondensate of terephthalic acid and nonanediamine).

Examples of the aliphatic polyamide include nylon 6 (a ring-opening polycondensate of ε-caprolactam), nylon 11 (a ring-opening polycondensate of undecane lactam), nylon 12 (a ring-opening polycondensate of lauryl lactam), nylon 66 (a condensed polymer of adipic acid and hexamethylenediamine), nylon 610 (a condensed polymer of sebacic acid and hexamethylenediamine), and nylon 612 (a condensed polymer of caprolactam (6 carbons) and lauryl lactam (12 carbon atoms)).

The resin particles according to the present exemplary embodiment preferably contain the polyamide compound (B) in an amount of 50 mass% or more, more preferably in an amount of 55 mass% or more and 95 mass% or less, still more preferably in an amount of 65 mass% or more and 93 mass% or less, and particularly preferably in an amount of 75 mass% or more and 91 mass% or less, with respect to the total amount of the resin particles.

The weight average molecular weight of the polyamide compound (B) is preferably 8,000 or more and 100,000 or less.

Unless otherwise specified, the weight average molecular weight of the polyamide compound (B) is a value measured according to the method for measuring the weight average molecular weight of the cellulose acylate (A).

From the viewpoint of improving the heat resistance and the flexibility of the resin particles, the polyamide compound (B) is preferably a polyamide compound (B1) having a constituent unit having 8 or more and 15 or less carbon atoms.

Here, the number of carbon atoms of the constituent unit refers to, in the case of a polyamide compound composed of one kind of monomer, the number of carbon atoms of the monomer. The number of carbon atoms of the constituent unit refers to, in the case of a polyamide compound composed of two or more kinds of monomers, the total number of carbon atoms in one monomer of respective molecules.

The polyamide compound (B 1) having a constituent unit having 8 or more and 15 or less carbon atoms is preferably at least one of nylon 11 (a ring-opening polycondensate of undecane lactam) or nylon 12 (a ring-opening polycondensate of lauryl lactam).

The resin particle according to the present exemplary embodiment preferably contains the polyamide compound (B1) having a constituent unit having 8 or more and 15 or less carbon atoms in an amount of 50 mass% or more with respect to the total amount of the resin particles.

The polyamide compound (B 1) having a constituent unit having 8 or more and 15 or less carbon atoms has a hydrocarbon group and an amide group having an appropriate length. Therefore, it is presumed that when the polyamide compound (B 1) is contained in an amount of 50 mass% or more with respect to the total amount of the resin particles, it is easier to form the above-mentioned "(1) state where the molecular chains of the resin contained in the resin particles have a spiral structure and the distance between the molecules is short". Therefore, the resin particles are more excellent in heat resistance.

From the viewpoint of further improving the heat resistance of the resin particles, the polyamide compound (B1) having a constituent unit having 8 or more and 15 or less carbon atoms is preferably contained in an amount of 55 mass% or more and 95 mass% or less, more preferably contained in an amount of 65 mass% or more and 93 mass% or less, and still preferably contained in an amount of 75 mass% or more and 91 mass% or less, with respect to the total amount of the resin particles.

### -Aliphatic Polyester Compound (C)-

Examples of an aliphatic polyester compound (C) include a polyhydroxy alkanoate and a polyester obtained by condensation polymerization of an aliphatic dihydric alcohol and an aliphatic dibasic acid (hereinafter, also referred to as "dibasic acid polyester").

The aliphatic polyester compound (C) is preferably contained in an amount of 50 mass% or more with respect to the total amount of the resin particles.

It is presumed that when the aliphatic polyester compound (C) is preferably contained in an amount of 50 mass% or more with respect to the total amount of the resin particles, it is easier to form the above-mentioned "(2) state where the distance between the resin molecules contained in the resin particles is long, but the molecules are firmly bonded to each other" and "(3) state where the molecular chains of the resin contained in the resin particles are intricately intertwined". Therefore, the resin particles are more excellent in heat resistance.

From the viewpoint of further improving the heat resistance of the resin particles, the aliphatic polyester compound (C) is preferably contained in an amount of 55 mass% or more and 95 mass% or less, more preferably contained in an amount of 65 mass% or more and 93 mass% or less, and still preferably contained in an amount of 75 mass% or more and 91 mass% or less, with respect to the total amount of the resin particles.

Among these, the aliphatic polyester compound (C) is preferably a polyhydroxy alkanoate from the viewpoint of making it easier to form a random coil and obtaining better heat resistance and flexibility of the resin particles.

### -Polyhydroxy alkanoate

Specific examples of the polyhydroxy alkanoate include a resin having a chemical structure represented by the general formula (2).

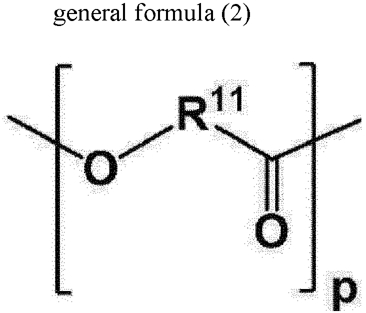

### (In the general formula (2), R¹¹ represents an alkylene group having 1 or more and 10 or less carbon atoms, p represents an integer of 2 or more.)

In the general formula (2), the alkylene group represented by R¹¹ is preferably an alkylene group having 2 or more and 6 or less carbon atoms from the viewpoint of further enhancing the heat resistance and the flexibility of the resin particles. The alkylene group represented by R¹¹ may be linear or branched, but is preferably branched from the viewpoint of further enhancing the heat resistance and the flexibility of the resin particles.

Here, in the general formula (2), R¹¹ representing an alkylene group indicates that 1) R¹¹ has a [OR¹¹-C(=O)-] structure representing the same alkylene group, and 2) R¹¹ has a plural [OR¹¹-C(= O)-] structures (that is, [OR^{11A}-C(=O)-] and [OR^{11B}-C(=O)-] structures) representing different alkylene groups (R¹¹ represents alkylene groups having different numbers of carbon numbers or different forms).

That is, the polyhydroxy alkanoate may be a homopolymer of one kind of hydroxy alkanoate (hydroxyalkanoic acid) or a copolymer of two or more kinds of hydroxy alkanoates (hydroxyalkanoic acids).

In the general formula (2), the upper limit of p is not particularly limited, but for example 20,000 or less. The range of p is preferably 500 or more and 10,000 or less, and more preferably 1,000 or more and 8,000 or less, from the viewpoint of further enhancing the heat resistance and the flexibility of the resin particles.

Examples of the hydroxyalkanoic acid that forms the polyhydroxy alkanoate include lactic acid, 2-hydroxybutyric acid, 3-hydroxybutyric acid, 4-hydroxybutyric acid, 2-hydroxy-3-methylbutyric acid, 2-hydroxy-3,3-dimethylbutyric acid, 3-hydroxyvaleric acid, 4-hydroxyvaleric acid, 5-hydroxyvaleric acid, 3-hydroxycaproic acid, 2-hydroxycaproic acid, 2-hydroxyisocaproic acid, 6-hydroxycaproic acid, 3-hydroxypropionic acid, 3-hydroxy-2,2-dimethylpropionic acid, 3-hydroxycaproic acid, and 2-hydroxy-n-octanoic acid.

Among these, the polyhydroxy alkanoate is preferably at least one of a homopolymer of a branched hydroxyalkanoic acid having 2 or more and 4 or less carbon atoms or a copolymer of a branched hydroxyalkanoic acid having 2 or more and 4 or less carbon atoms and a branched hydroxyalkanoic acid having 5 or more and 7 or more carbon atoms (the number of carbon atoms is the number including the carbon atoms of the carboxy group), from the viewpoint of making it easier to form a random coil and increasing the heat resistance and the flexibility of the resin particles. In particular, a copolymer of 3-hydroxybutyric acid and 3-hydroxyhexanoic acid (a copolymer of 3-hydroxybutyrate and 3-hydroxyhexanoate) is more preferred.

When the polyhydroxy alkanoate is a copolymer of 3-hydroxybutyrate and 3-hydroxyhexanoate, the copolymerization ratio of 3-hydroxyhexanoate to the copolymer of 3-hydroxybutyrate and 3-hydroxyhexanoate is preferably 3 mol% or more and 20 mol% or less, preferably 4 mol% or more and 15 mol% or less, and more preferably 5 mol% or more and 12 mol% or less.

The method of measuring the copolymerization ratio of 3-hydroxyhexanoate to the copolymer of 3-hydroxybutyrate and 3-hydroxyhexanoate is as follows.

Using H¹-NMR, the hexanoate ratio is calculated from the integrated value of the peaks derived from the hexanoate terminal and the butyrate terminal.

The weight average molecular weight (Mw) of the polyhydroxy alkanoate is 10,000 or more and 1,000,000 or less (preferably 50,000 or more and 800,000 or less, and more preferably 100,000 or more and 600,000 or less).

When the weight average molecular weight (Mw) of the polyhydroxy alkanoate is within the above range, resin particles excellent in heat resistance and flexibility can be easily obtained.

The weight average molecular weight (Mw) of polyhydroxy alkanoate is a value measured by gel permeation chromatography (GPC). Specifically, the molecular weight measurement by using GPC is performed with a chloroform solvent using HPLC1100 manufactured by Tosoh Corporation as a measurement device and a column TSKgel GMHHR-M+TSKgel GMHHR-M (7.8 mm I. D. 30 cm) manufactured by Tosoh Corporation. Then, the weight average molecular weight is calculated from the measurement result using a molecular weight calibration curve prepared using a monodispersed polystyrene standard sample.

Specific examples of the polyhydroxy alkanoate will be shown below, but the present invention is not limited to this.

| | Compound name | Product name | Manufacturer |
|---|---|---|---|
| PHA-1 | Poly(3-hydroxybutyrate-co-3-hydroxyhexanoate) (copolymer of 3-hydroxybutyric acid and 3-hydroxycaproic acid) | Aonilex | KANEKA |
| PHA-2 | Polylactic acid | TERRAMAC TE2000 | UNITIKA |
| PHA-3 | Poly(3-hydroxybutyrate-co-3-hydroxyvariate) (copolymer of 3-hydroxybutyric acid and 3-hydroxyvaleric acid) | Biopol | AstraZeneca |
| PHA-4 | Poly(3-hydroxybutyrate) (homopolymer of 3-hydroxybutyric acid) | Biopol | AstraZeneca |

### -Dibasic acid polyester

The dibasic acid polyester is a polyester obtained by condensation polymerization of an aliphatic dihydric alcohol and an aliphatic dibasic acid.

Examples of the aliphatic dihydric alcohol include a linear or branched divalent alcohol having 2 or more and 10 or less carbon atoms.

Specific examples of the aliphatic dihydric alcohol include ethylene glycol, diethylene glycol, triethylene glycol, polyethylene glycol, propylene glycol, dipropylene glycol, 1,3-butanediol, 1,4-butanediol, 3-methyl-1,5-pentanediol, 1,6-hexanediol, 1,9-nonanediol, and neopentyl glycol. The aliphatic dihydric alcohol may be used alone or in combination of two or more thereof.

The aliphatic dihydric alcohol is preferably 1,4-butanediol.

Examples of the aliphatic dibasic acid include a linear or branched divalent carboxylic acid having 2 or more and 15 or less carbon atoms.

Specific examples of the aliphatic dibasic acid include oxalic acid, succinic acid, glutaric acid, adipic acid, pimelic acid, suberic acid, azelaic acid, sebacic acid, undecanedioic acid, and dodecanedioic acid. The aliphatic dibasic acid may be used alone or in combination of two or more thereof.

The aliphatic dibasic acid is preferably at least one of succinic acid or adipic acid.

The dibasic acid polyester is preferably, for example, at least one of polybutylene succinate or polybutylene succinate adipate.

The weight average molecular weight (Mw) of the dibasic acid polyester is preferably 8,000 or more and 100,000 or less (preferably 15,000 or more and 70,000 or less, and more preferably 20,000 or more and 60,000 or less).

Unless otherwise specified, the weight average molecular weight of the dibasic acid polyester is a value measured according to the method for measuring the weight average molecular weight of the polyhydroxy alkanoate.

### -Oxidative Biodegradable Resin-

The oxidative biodegradable resin refers to a resin containing an oxidative decomposition agent.

The oxidative decomposition agent refers to a substance that can oxidatively decompose a resin to reduce the molecular weight to the extent that it can be decomposed by microorganisms.

Examples of the oxidative decomposition agent include a carboxylic acid metal salt, a hydroxycarboxylic acid, a transition metal compound, a rare earth compound, and an aromatic ketone.

Examples of the carboxylic acid metal salt include a metal salt of an aliphatic carboxylic acid having 10 or more and 20 or less carbon atoms, and a metal stearic acid salt.

Examples of the hydroxycarboxylic acid include monohydroxytricarboxylic acid, trihydroxyglutarate, polyhydroxydicarboxylic acid, dihydroxydicarboxylic acid, tartronic acid, monohydroxydicarboxylic acid, polyhydroxymonocarboxylic acid, glyoxylic acid, and dihydroxymonocarboxylic acid.

Examples of the transition metal compound include a cobalt or magnesium salt, and more specifically, a cobalt or magnesium salt of an aliphatic carboxylic acid (12 to 20 carbon atoms).

Examples of the rare earth compound include rare earth oxides, rare earth hydroxides, rare earth sulfates, rare earth nitrates, rare earth acetates, rare earth chlorides, and rare earth carboxylic acid salts.

Examples of the aromatic ketone include benzophenone, anthraquinone, anthrone, acetylbenzophenone, and 4-octylbenzophenone.

The oxidative decomposition agent may be used alone or in combination of two or more thereof.

The oxidative decomposition agent is not particularly limited, and examples thereof include those disclosed in JP-A-2011-212170.

Specifically, as the oxidative decomposition agent, for example, the trade name "P-life" (manufactured by P-Life Japan, Inc.) can be used.

The resin to which the oxidative decomposition agent is added is not particularly limited, and is preferably a polyolefin, and more preferably at least one of a polyethylene or a polypropylene.

The content of the oxidative decomposition agent with respect to the total amount of the oxidative biodegradable resin is preferably 1 mass% or more and 50 mass% or less, more preferably 1.5 mass% or more and 30 mass% or less, and still more preferably 3 mass% or more and 25 mass% or less.

The oxidative biodegradable resin is preferably contained in an amount of 50 mass% or more, more preferably contained in an amount of 70 mass% or more and 100 mass% or less, still more preferably contained in an amount of 80 mass% or more and 100 mass% or less, and particularly preferably contained in an amount of 90 mass% or more and 100 mass% or less, with respect to the total amount of the resin particles.

The resin component contained in the resin particle according to the present exemplary embodiment may contain a resin other than the biodegradable resin. However, when other resins are contained, the content of other resins with respect to the total amount of the resin particles is preferably 20 mass% or less, more preferably 10 mass% or less, and still more preferably 5 mass% or less.

Examples of other resins include thermoplastic resins known in the related art. Specific examples thereof include: a polycarbonate resin; a polypropylene resin; a polyester resin; a polyolefin resin; a polyester carbonate resin; a polyphenylene ether resin; a polyphenylene sulfide resin; a polysulfone resin; a polyether sulfone resin; a polyarylene resin; a polyetherimide resin; a polyacetal resin; a polyvinyl acetal resin; a polyketone resin; a polyetherketone resin; a polyetheretherketone resin; a polyarylketone resin; a polyethernitrile resin; a liquid crystal resin; a polybenzimidazole resin; a polyparavanic acid resin; a vinyl polymer or copolymer obtained by polymerizing or copolymerizing one or more vinyl monomers selected from the group consisting of an aromatic alkenyl compound, a methacrylate ester, an acrylate ester, and a vinyl cyanide compound; a diene-aromatic alkenyl compound copolymer; a vinyl cyanide-diene-aromatic alkenyl compound copolymer; an aromatic alkenyl compound-diene-vinyl cyanide-N-phenylmaleimide copolymer; a vinyl cyanide-(ethylene-diene-propylene(EPDM))-aromatic alkenyl compound copolymer; a vinyl chloride resin; and a chlorinated vinyl chloride resin. These resins may be used alone or in combination of two or more thereof.

### -Aromatic Compound (D)-

The aromatic compound (D) is an aromatic compound having a long-chain aliphatic group having 8 or more and 20 or less carbon atoms and having at least one of a phenolic hydroxy group or a monoglycidyl ether group directly bonded to the aromatic group.

That is, the aromatic compound (D) is a compound having a long-chain aliphatic group having 8 or more carbon atoms and 20 or less carbon atoms, and having at least one of a phenolic hydroxy group or a monoglycidyl ether group.

Here, examples of the long-chain aliphatic group having 8 or more carbon atoms and 20 or less carbon atoms include saturated aliphatic groups (alkyl group) and unsaturated aliphatic groups (alkenyl group and alkynyl group) having 8 or more and 20 or less (preferably 10 or more and 20 or less) carbon atoms. The aliphatic group may be linear, branched or cyclic, and is preferably linear or branched, and more preferably linear.

Examples of the aromatic compound (D) include a compound in which a phenolic hydroxy group is substituted, together with a long-chain aliphatic group, with a monocyclic ring, a condensed ring (polycyclic ring with two or more aromatic rings), a polynuclear ring (polycyclic ring in which aromatic rings are bonded via a carbon-carbon bond), or a heterocyclic ring (a monocyclic ringcle of a heterocyclic ring, a condensed ring including a heterocyclic ring, polynuclear ring including a heterocyclic ring, etc.).

Specific examples of the aromatic compound (D) include a cardanol compound, a phenalkamine compound, a phenol resin, a phenol novolac type epoxy resin, a phenol resol type epoxy resin, a phenol-modified palm oil, a phenol-modified soybean oil, and a phenol-modified flaxseed oil.

The resin particle according to the present exemplary embodiment preferably contains the aromatic compound (D) in an amount of 3 mass% or more and less than 50 mass% with respect to the total amount of the resin particles.

It is presumed that when the aromatic compound (D) is contained in an amount of 3 mass% or more and less than 50 mass% with respect to the total amount of the resin particles, it is easier to form the above-mentioned "(2) state where the distance between the resin molecules contained in the resin particles is long, but the molecules are firmly bonded to each other". Therefore, the resin particles are more excellent in heat resistance and flexibility.

From the viewpoint of further improving the heat resistance and the flexibility of the resin particles, the aromatic compound (D) is preferably contained in an amount of 4 mass% or more and 30 mass% or less, more preferably contained in an amount of 6 mass% or more and 25 mass% or less, and still preferably contained in an amount of 8 mass% or more and 20 mass% or less, with respect to the total amount of the resin particles.

The aromatic compound (D) is preferably a cardanol compound (D1).

It is presumed that since the cardanol compound (D1) has an appropriate molecular size, it is easier to bind molecules contained in resin particles and it is easier to form the above-mentioned "(2) state where the distance between the resin molecules contained in the resin particles is long, but the molecules are firmly bonded to each other".

The cardanol compound (D1) refers to a component contained in a naturally-derived compound made from cashew (for example, a compound represented by the following structural formulas (d-1) to (d-4)) or a derivative of the component.

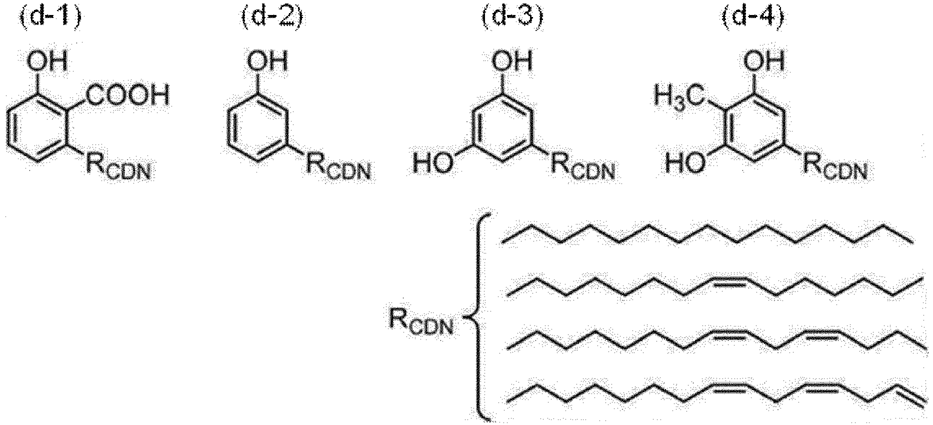

The cardanol compound (D1) may be a mixture of a naturally-derived compound made from cashew (hereinafter, also referred to as "cashew-derived mixture").

The cardanol compound (D1) may be a derivative of the cashew-derived mixture. Examples of the derivative of the cashew-derived mixture include the following mixtures and simple substances.
· Mixture prepared by adjusting the composition ratio of each component in the cashew-derived mixture
· Simple substance obtained by isolating only a specific component from the cashew-derived mixture
· Mixture containing a modified product obtained by modifying components in the cashew-derived mixture
· Mixture containing a polymer obtained by polymerizing components in the cashew-derived mixture
· Mixture containing a modified polymer obtained by modifying and polymerizing components in the cashew-derived mixture
· Mixture containing a modified product obtained by further modifying components in the mixture whose composition ratio is adjusted
· Mixture containing a polymer obtained by further polymerizing components in the mixture whose composition ratio is adjusted
· Mixture containing a modified polymer obtained by further modifying and polymerizing components in the mixture whose composition ratio is adjusted
· Modified product obtained by further modifying the isolated simple substance
· Polymer obtained by further polymerizing the isolated simple substance
· Modified polymer obtained by further modifying and polymerizing the isolated simple substance

Here, the simple substance also includes multimers such as a dimer and a trimer.

The cardanol compound (D1) is preferably at least one compound selected from the group consisting of a compound represented by a general formula (CDN1) and a polymer obtained by polymerizing the compound represented by the general formula (CDN1), from the viewpoint of improving the biodegradation rate of the resin molded product.

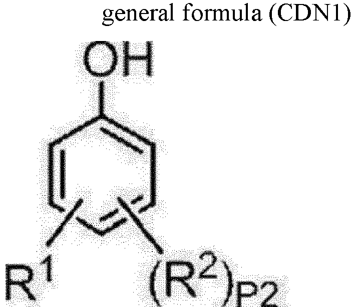

In the general formula (CDN1), R¹ represents an alkyl group which may have a substituent or an unsaturated aliphatic group which has a double bond and may have a substituent. R² represents a hydroxy group, a carboxy group, an alkyl group which may have a substituent, or an unsaturated aliphatic group which has a double bond and may have a substituent. P2 represents an integer of 0 or more and 4 or less. When P2 is 2 or more, plural R²s may be the same group or different groups.

In the general formula (CDN1), the alkyl group which may have a substituent and represented by R¹ is preferably an alkyl group having 3 or more and 30 or less carbon atoms, more preferably an alkyl group having 5 or more and 25 or less carbon atoms, and still more preferably an alkyl group having 8 or more and 20 or less carbon atoms.

Examples of the substituent include a hydroxy group; a substituent containing an ether bond such as an epoxy group and a methoxy group; and a substituent containing an ester bond such as an acetyl group and a propionyl group.

Examples of the alkyl group which may have a substituent include a pentadecane-1-yl group, a heptane-1-yl group, an octane-1-yl group, a nonane-1-yl group, a decane-1-yl group, an undecane-1-yl group, a dodecane-1-yl group, and a tetradecane-1-yl group.

In the general formula (CDN1), the unsaturated aliphatic group which has a double bond and may have a substituent and represented by R¹ is preferably an unsaturated aliphatic group having 3 or more and 30 or less carbon atoms, more preferably an unsaturated aliphatic group having 5 or more and 25 or less carbon atoms, and still more preferably an unsaturated aliphatic group having 8 or more and 20 or less carbon atoms.

The number of the double bond contained in the unsaturated aliphatic group is preferably 1 or more and 3 or less.

Examples of the substituent include those listed as the substituent of the above alkyl group.

Examples of the unsaturated aliphatic group which has a double bond and may have a substituent include a pentadeca-8-en-1-yl group, a pentadeca-8,11-diene-1-yl group, a pentadeca-8,11,14-trien-1-yl group, a pentadeca-7-en-1-yl group, a pentadeca-7,10-diene-1-yl group, and a pentadeca-7,10,14-trien-1-yl group.

In the general formula (CDN1), R¹ is preferably a pentadeca-8-ene-1-yl group, a pentadeca-8,11-diene-1-yl group, a pentadeca-8,11,14-triene-1-yl group, a pentadeca-7-ene-1-yl group, a pentadeca-7,10-diene-1-yl group, and a pentadeca-7,10,14-triene-1-yl group.

In the general formula (CDN1), similarly, preferred examples of the alkyl group which may have a substituent and the unsaturated aliphatic group which has a double bond and may have a substituent, which are represented by R², include those listed as the alkyl group which may have a substituent and the unsaturated aliphatic group which has a double bond and may have a substituent, which are represented by R¹.

The compound represented by the general formula (CDN1) may be further modified. For example, the compound may be epoxidized. Specifically, the compound may be a compound having a structure in which the hydroxy group of the compound represented by the general formula (CDN1) is substituted with the following group (EP), i.e., a compound represented by the following general formula (CDN1-e).

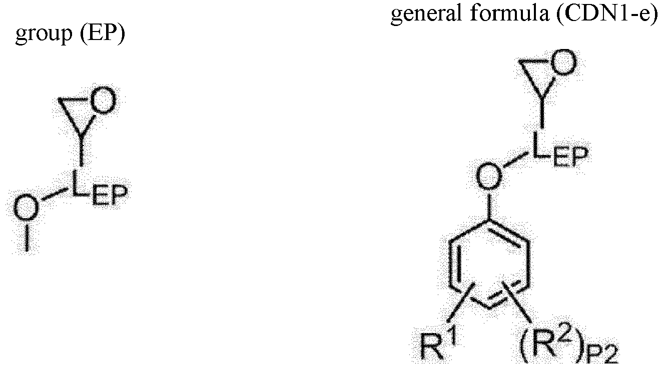

In the group (EP) and the general formula (CDN1-e), L_{EP} represents a single bond or a divalent linking group. In the general formula (CDN1-e), R¹, R² and P2 have the same meaning with R¹, R² and P2 in the general formula (CDN1), respectively.

In the group (EP) and the general formula (CDN1-e), examples of the divalent linking group represented by L_{EP} include an alkylene group which may have a substituent (preferably an alkylene group having 1 or more and 4 or less carbon atoms, and more preferably an alkylene group having 1 carbon atom), and a -CH₂CH₂OCH₂CH₂- group.

Examples of the above substituent include those listed as the substituent of R¹ in the general formula (CDN1).

L_{EP} is preferably a methylene group.

The polymer obtained by polymerizing the compound represented by the general formula (CDN1) refers to a polymer obtained by polymerizing at least two compounds represented by the general formula (CDN1) with or without a linking group.

Examples of the polymer obtained by polymerizing the compound represented by the general formula (CDN1) include a compound represented by the following general formula (CDN2).

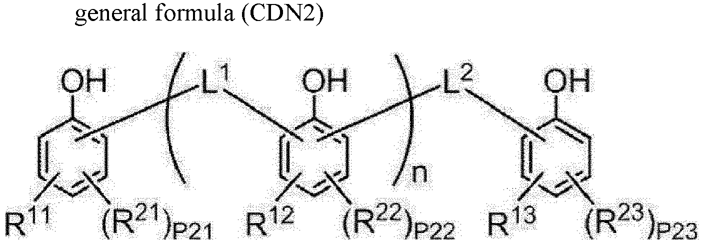

In the general formula (CDN2), R¹¹, R¹² and R¹³ each independently represent an alkyl group which may have a substituent or an unsaturated aliphatic group which has a double bond and may have a substituent. R²¹, R²² and R²³ each independently represent a hydroxy group, a carboxy group, an alkyl group which may have a substituent, or an unsaturated aliphatic group which has a double bond and may have a substituent. P21 and P23 each independently represent an integer of 0 or more and 3 or less, and P22 represents an integer of 0 or more and 2 or less. L¹ and L² each independently represent a divalent linking group. n represents an integer of 0 or more and 10 or less. Plural R²¹s when P21 is 2 or more, plural R²²s when P22 is 2 or more, and plural R²³s when P23 is 2 or more may be the same group or different groups, separately. Plural R¹²s, R²²s, and L¹s when n is 2 or more may be the same group or different groups separately, and plural P22s when n is 2 or more may be the same group or different groups.

In the general formula (CDN2), preferred examples of the alkyl group which may have a substituent and the unsaturated aliphatic group which has a double bond and may have a substituent, which are represented by R¹¹, R¹², R¹³, R²¹, R²² and R²³, include those listed as R¹ in the general formula (CDN1).

In the general formula (CDN2), examples of the divalent linking group represented by L¹ and L² include an alkylene group which may have a substituent (preferably an alkylene group having 2 or more and 30 or less carbon atoms, and more preferably an alkylene group having 5 or more and 20 or less carbon atoms).

Examples of the above substituent include those listed as the substituent of R¹ in the general formula (CDN1).

In the general formula (CDN2), n is preferably 1 or more and 10 or less, and more preferably 1 or more and 5 or less.

The compound represented by the general formula (CDN2) may be further modified. For example, the compound may be epoxidized. Specifically, the compound may be a compound having a structure in which the hydroxy group of the compound represented by the general formula (CDN2) is substituted with the group (EP), i.e., a compound represented by the following general formula (CDN2-e).

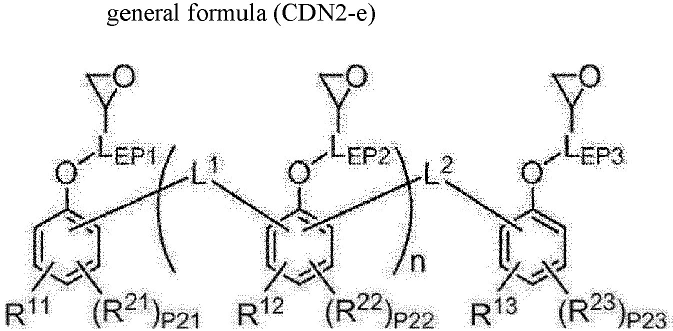

In the general formula (CDN2-e), R¹¹, R¹², R¹³, R²¹, R²², R²³, P21, P22, P23, L¹, L² and n have the same meaning with R¹¹, R¹², R¹³, R²¹, R²², R²³, P21, P22, P23, L¹, L² and n in the general formula (CDN2), respectively.

In the general formula (CDN2-e), L_{EP1}, L_{EP2} and L_{EP3} each independently represent a single bond or a divalent linking group. When n is 2 or more, plural L_{EP2}s may be the same group or different groups.

In the general formula (CDN2-e), preferred examples of the divalent linking group represented by L_{EP1}, L_{EP2} and L_{EP3} include those listed as the divalent linking group represented by L_{EP} in the general formula (CDN1-e).

The polymer obtained by polymerizing the compound represented by the general formula (CDN1) may be, for example, a polymer obtained by three-dimensionally crosslinking and polymerizing at least three compounds represented by the general formula (CDN1) with or without a linking group. Examples of the polymer obtained by three-dimensionally crosslinking and polymerizing the compounds represented by the general formula (CDN1) include a compound represented by the following structural formula.

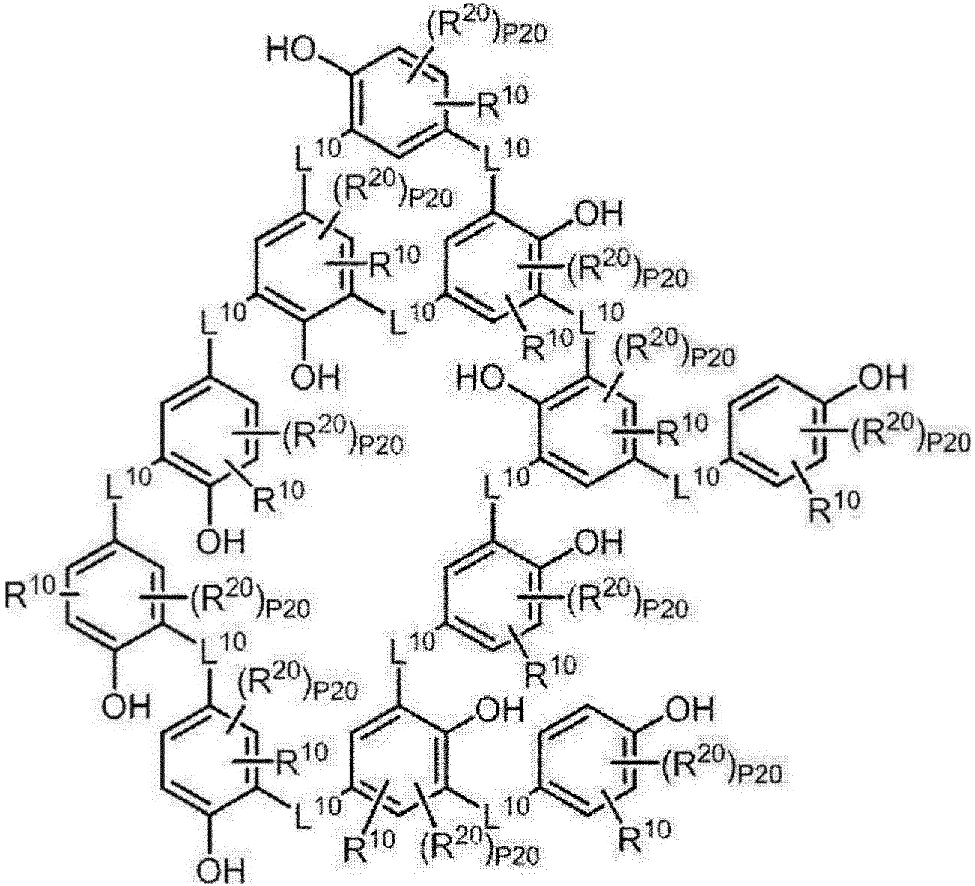

In the above structural formula, R¹⁰, R²⁰ and P20 have the same meaning with R¹, R² and P2 in the general formula (CDN1), respectively. L¹⁰ represents a single bond or a divalent linking group. Plural R¹⁰s, R²⁰s and L¹⁰s may be the same group or different groups, separately. Plural P20s may be the same number or different numbers.

In the above structural formula, examples of the divalent linking group represented by L¹⁰ include an alkylene group which may have a substituent (preferably an alkylene group having 2 or more and 30 or less carbon atoms, and more preferably an alkylene group having 5 or more and 20 or less carbon atoms).

Examples of the above substituent include those listed as the substituent of R¹ in the general formula (CDN1).

The compound represented by the above structural formula may be further modified, for example, epoxidized. Specifically, the compound may be a compound having a structure in which the hydroxy group of the compound represented by the above structural formula is substituted with the group (EP), for example, a compound represented by the following structural formula, that is, a polymer obtained by three-dimensionally crosslinking and polymerizing the compound represented by the general formula (CDN1-e).

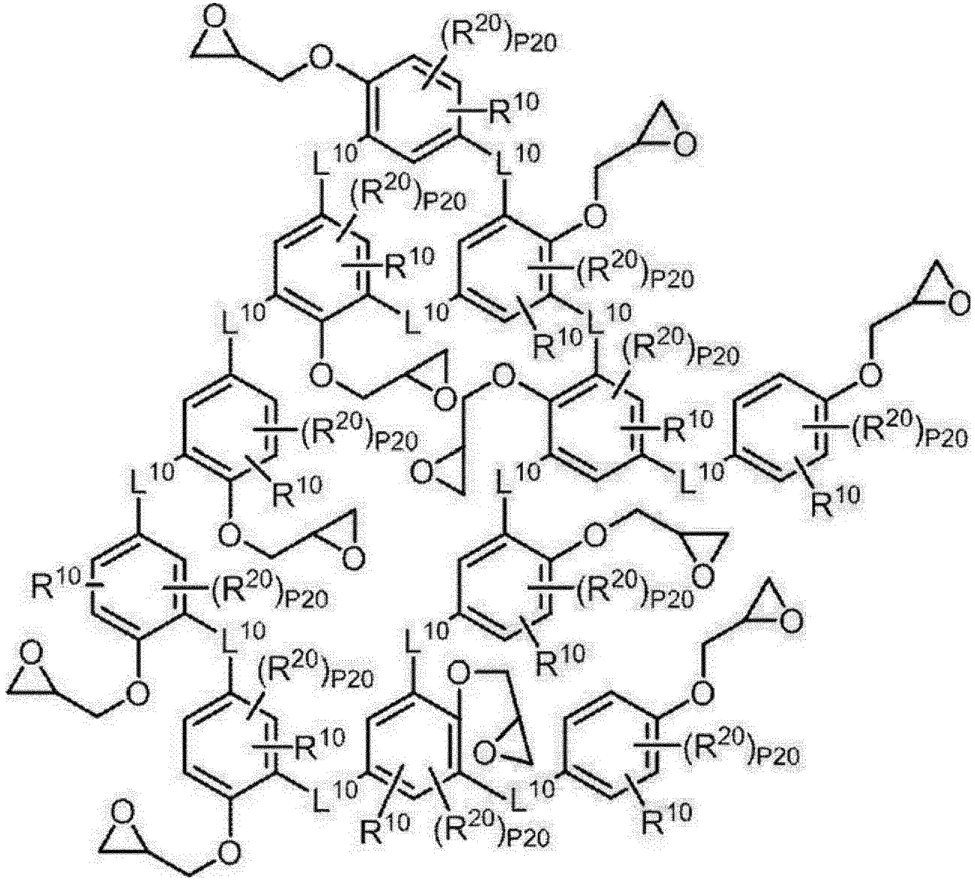

In the above structural formula, R¹⁰, R²⁰ and P20 have the same meaning with R¹, R² and P2 in the general formula (CDN1-e), respectively. L¹⁰ represents a single bond or a divalent linking group. Plural R¹⁰s, R²⁰s and L¹⁰s may be the same group or different groups, separately. Plural P20s may be the same number or different numbers.

In the above structural formula, examples of the divalent linking group represented by L¹⁰ include an alkylene group which may have a substituent (preferably an alkylene group having 2 or more and 30 or less carbon atoms, and more preferably an alkylene group having 5 or more and 20 or less carbon atoms).

Examples of the above substituent include those listed as the substituent of R¹ in the general formula (CDN1).

As the cardanol compound (D1), a commercially available product may be used. Examples of the commercially available product include: NX-2024, Ultra LITE 2023, NX-2026, GX-2503, NC-510, LITE 2020, NX-9001, NX-9004, NX-9007, NX-9008, NX-9201, and NX-9203, manufactured by Cardolite Corporation; and LB-7000, LB-7250, and CD-5L, manufactured by Tohoku Chemical Industry Co., Ltd. Examples of the commercially available product of a cardanol compound having an epoxy group include NC-513, NC-514S, NC-547, LITE 513E, and Ultra LTE 513 manufactured by Cardolite Corporation.

The cardanol compound (D1) preferably has a hydroxyl value of 100 mgKOH/g or more, more preferably 120 mgKOH/g or more, and still more preferably 150 mgKOH/g or more, from the viewpoint of improving the biodegradation rate of the resin molded product. The hydroxyl value of the cardanol compound (D1) is measured according to Method A of ISO14900.

When a cardanol compound (D1) having an epoxy group is used as the cardanol compound (D1), an epoxy equivalent is preferably 300 or more and 500 or less, more preferably 350 or more and 480 or less, and still more preferably 400 or more and 470 or less, from the viewpoint of improving the transparency of the resin molded product. The epoxy equivalent of the cardanol compound (D1) having an epoxy group is measured according to ISO3001.

The molecular weight of the cardanol compound (D1) is preferably 250 or more and 1000 or less, more preferably 280 or more and 800 or less, and still more preferably 300 or more and 500 or less, from the viewpoint of improving the heat resistance and the flexibility of the resin particles.

Unless otherwise specified, the molecular weight of the cardanol compound (D1) is a value measured according to the method for measuring the weight average molecular weight of the cellulose acylate (A).

The cardanol compound (D1) may be used alone or in combination of two or more thereof.

### -Ester Compound (E)-

The ester compound (E) may be any of a monoester, a diester, a triester and a polyester.

Examples of the ester compound (E) include aliphatic monocarboxylic acid esters (acetate ester, etc.), aliphatic dicarboxylic acid esters (succinate ester, adipate ester, azelate ester, sebacate ester, stearate ester, etc.), aliphatic tricarboxylic acid esters (citrate ester, isocitrate ester, etc.), epoxidized fatty acid esters (epoxidized soybean oil, epoxidized flaxseed oil, epoxidized rapeseed fatty acid isobutyl, and epoxidized fatty acid 2-ethylhexyl), fatty acid methyl esters, and sucrose esters.

The ester compound (E) may be acylated with an alkylcarboxylic acid anhydride (for example, a linear or branched alkylcarboxylic acid anhydride having 2 or more and 6 or less (preferably 2 or more and 3 or less) carbon atoms such as acetic anhydride, propionic anhydride, butyric anhydride, and valeric anhydride).

Preferred examples of the ester compound (E) include aliphatic dicarboxylic acid esters (particularly adipate ester and sebacate ester) and aliphatic tricarboxylic acid esters (particularly citrate ester).

Specific examples of the adipate ester include an adipic acid diester represented by the following general formula (AE) and an adipic acid polyester represented by the following general formula (APE).

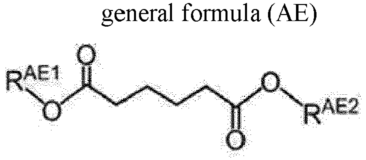

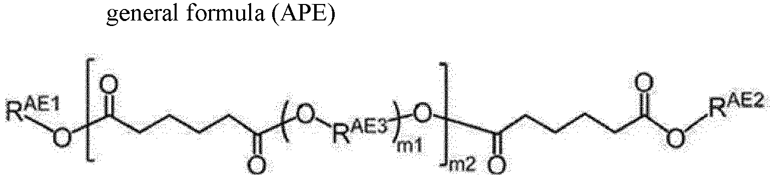

In the general formula (AE), R^{AE1} and R^{AE2} each independently represent an alkyl group or a polyoxyalkyl group [-(CₓH₂ₓ-O)_{y}-R^{A1}] (where R^{A1} represents an alkyl group, x represents an integer of 1 or more and 10 or less, and y represents an integer of 1 or more and 10 or less).

In the general formula (APE), R^{AE1} and R^{AE2} each independently represent an alkyl group or a polyoxyalkyl group [-(CₓH₂ₓ-O)y-R^{A1}] (where R^{A1} represents an alkyl group, x represents an integer of 1 or more and 10 or less, and y represents an integer of 1 or more and 10 or less), and R^{AE3} represents an alkylene group. m1 represents an integer of 1 or more and 10 or less, and m2 represents an integer of 1 or more and 20 or less.

In the general formulas (AE) and (APE), the alkyl group represented by R^{AE1} and R^{AE2} is preferably an alkyl group having 1 or more and 12 or less carbon atoms, more preferably an alkyl group having 4 or more and 10 or less carbon atoms, and still more preferably an alkyl group having 8 carbon atoms. The alkyl group represented by R^{AE1} and R^{AE2} may be linear, branched or cyclic, and is preferably linear or branched.

In the general formulas (AE) and (APE), in the polyoxyalkyl group [-(CₓH₂ₓ-O)_{y}-R^{A1}] represented by R^{AE1} and R^{AE2}, the alkyl group represented by R^{A1} is preferably an alkyl group having 1 or more and 6 or less carbon atoms, and more preferably an alkyl group having 1 or more and 4 or less carbon atoms. The alkyl group represented by R^{A1} may be linear, branched or cyclic, and is preferably linear or branched.

In the general formula (APE), the alkylene group represented by R^{AE3} is preferably an alkylene group having 1 or more and 6 or less carbon atoms, and more preferably an alkylene group having 1 or more and 4 or less carbon atoms. The alkylene group may be linear, branched or cyclic, and is preferably linear or branched.

In the general formula (APE), m1 is preferably an integer of 1 or more and 5 or less, and m2 is preferably an integer of 1 or more and 10 or less.

In the general formulas (AE) and (APE), the group represented by each reference numeral may be substituted with a substituent. Examples of the substituent include an alkyl group, an aryl group, and a hydroxy group.

As the adipate ester, a mixture of the adipate ester and other components may be used. Examples of commercially available products of the mixture include Daifatty 101 manufactured by DAH4ACHI CHEMICAL INDUSTRY CO., LTD.

Examples of the sebacate ester include alkyl esters of sebacic acid having 1 or more and 12 or less (preferably 1 or more and 8 or less) carbon atoms.

Examples of the citrate ester include alkyl esters of citric acid having 1 or more and 12 or less (preferably 1 or more and 8 or less) carbon atoms. The citrate ester may be a citrate ester acylated with an alkylcarboxylic acid anhydride (for example, a linear or branched alkylcarboxylic acid anhydride having 2 or more and 6 or less (preferably 2 or more and 3 or less) carbon atoms such as acetic anhydride, propionic anhydride, butyric anhydride, and valeric anhydride).

The molecular weight (or weight average molecular weight) of the ester compound (E) is preferably 130 or more and 2,000 or less, more preferably 150 or more and 1,500 or less, still more preferably 170 or more and 1,000 or less, and most preferably 200 or more and less than 500.

Here, among the ester compound (E), an ester compound having a molecular weight of 200 or more and less than 500 is referred to as an ester compound (E1).

Unless otherwise specified, the molecular weight of the ester compound (E) is a value measured according to the method for measuring the weight average molecular weight of the cellulose acylate (A).

The resin particle according to the present exemplary embodiment preferably contains the ester compound (E1) having a molecular weight of 200 or more and less than 500 in an amount of 5 mass% or more and less than 30 mass% with respect to the total amount of the resin particles.

It is presumed that when the ester compound (E1) is contained in an amount of 5 mass% or more and less than 30 mass% with respect to the total amount of the resin particles, it is easy to form the above-mentioned "(2) state where the distance between the resin molecules contained in the resin particles is long, but the molecules are firmly bonded to each other".

From the viewpoint of further improving the heat resistance of the resin particles, the ester compound (E) is preferably contained in an amount of 10 mass% or more and 25 mass% or less, and more preferably contained in an amount of 13 mass% or more and 22 mass% or less, with respect to the total amount of the resin particles.

The ester compound (E1) is preferably at least one of an adipate ester or a citrate ester.

It is presumed that since the adipate ester has an appropriate molecular size, it is easier to bind molecules contained in resin particles and it is easier to form the above-mentioned "(2) state where the distance between the resin molecules contained in the resin particles is long, but the molecules are firmly bonded to each other".

It is presumed that since the citrate ester has good compatibility with resin components, it is easier to form the above-mentioned "(2) state where the distance between the resin molecules contained in the resin particles is long, but the molecules are firmly bonded to each other".

Here, when the cellulose acylate (A) is applied as the resin component, it is particularly preferable that the resin particle contains the cellulose acylate (A) and at least one of the aromatic compound (D) or the ester compound (E) as a plasticizer.

The cellulose acylate (A) is less likely to hydrolyze by itself, and is difficult to mold because of being inferior in flexibility, so that it is preferable to add a plasticizer to the cellulose acylate (A) in order to impart flexibility. However, the cellulose acylate (A) to which the plasticizer is added is easily hydrolyzed. Therefore, coating the resin particle containing the cellulose acylate (A) and the plasticizer with the second layer containing a hydrophobic compound is effective in imparting hydrolysis resistance while ensuring biodegradability.

On the other hand, in order to slow down the initial biodegradation rate, it is preferable that the resin particle is firmly coated with the second layer containing a hydrophobic compound.

In this regard, when at least one of the aromatic compound (D) or the ester compound (E) is selected as the plasticizer to be contained in the resin particle, the first layer containing a cationic resin, which functions as an adhesive layer between the surface of the resin particle and the second layer containing a hydrophobic compound, is more firmly coated on the surface of the resin particle. As a result, the resin particle can be more firmly coated with the second layer containing a hydrophobic compound, and high hydrolysis resistance can be imparted to the resin particle while ensuring biodegradability. The reasons are presumed as follows.

Since the aromatic compound (D) has a benzene ring, the benzene ring attracts electrons and the OH group becomes acidic. Therefore, the reactivity with a cationic compound is high. Further, since the ester compound (E) is a compound that is acidic, it has high reactivity with a cationic compound. As a result, the first layer containing a cationic resin is more firmly coated on the surface of the resin particle.

Therefore, it is presumed that the resin particle can be more firmly coated with the second layer containing a hydrophobic compound, and high hydrolysis resistance can be imparted to the biodegradable resin particle while ensuring biodegradability. As a result, the biodegradable resin particle has excellent hydrolysis resistance while ensuring biodegradability.

In particular, when cellulose acetate propionate is applied as the cellulose acylate (A), it is preferable to apply a cardanol compound as the aromatic compound (D) to the plasticizer from the viewpoint of improving the hydrolysis resistance while ensuring the biodegradability.

From the same viewpoint, when cellulose acetate propionate is applied as the cellulose acylate (A), it is preferable to apply the ester compound (E) to the plasticizer.

### -Other Components-

The resin composition according to the present exemplary embodiment may contain other components.

Examples of other components include antioxidants, plasticizers, flame retardants, compatibilizers, mold release agents, light-resistant agents, weatherproofing agents, colorants, pigments, modifiers, drip inhibitors, antistatic agents, hydrolysis inhibitors, fillers, reinforcing agents (glass fiber, carbon fiber, talc, clay, mica, glass flakes, milled glass, glass beads, crystalline silica, alumina, silicon nitride, aluminum nitride, boron nitride, etc.), acid acceptors for preventing acid release (oxides such as magnesium oxide and aluminum oxide; metal hydroxides such as magnesium hydroxide, calcium hydroxide, aluminum hydroxide and hydrotalcite; calcium carbonate; talcite; etc.), and reactive trapping agents (epoxy compounds, acid anhydride compounds, carbodiimide, etc.).

The content of the other components is preferably 0 mass% or more and 5 mass% or less with respect to the total amount of the resin composition. Here, "0 mass%" means that the resin particles do not contain other components.

The other components preferably contain an antioxidant from the viewpoints of achieving a high melting point and improving the heat resistance of the resin particles.

Examples of the antioxidant include a compound having a phenolic hydroxy group, ascorbic acid and a derivative thereof, and a phosphate ester compound.

### · Compound having phenolic hydroxy group

Examples of the compound having a phenolic hydroxy group include dibutylhydroxytoluene, butylhydroxyanisole, propyl gallate, tocopherol, chloroformate catechin, and a hindered phenol compound.

Here, examples of the hindered phenol compound include triethylene glycol bis[3-(3-t-butyl-5-methyl-4-hydroxyphenyl) propionate], pentaerythrityl tetrakis[3-(3,5-di-t-butyl-4-hydroxyphenyl) propionate], and 2,2-thio-diethylene bis[3-(3,5-di-t-butyl-4-hydroxyphenyl) propionate].

### · Ascorbic acid and derivative thereof

Examples of the scorbic acid and a derivative thereof include L-ascorbic acid, sodium L-ascorbate, potassium L-ascorbate, calcium L-ascorbate, L-ascorbic acid phosphate ester, a magnesium salt of L-ascorbic acid phosphate ester, L-ascorbic acid sulfate ester, a disodium salt of L-ascorbic acid sulfate ester, L-ascorbic acid stearate ester, L-ascorbic acid 2-glucoside ester, L-ascorbic acid palmitate, and tetraisopalmitate L-ascorbyl.

### · Phosphate ester compound

Examples of the phosphate ester compound include tris(2,4-di-t-butylphenyl) phosphite, 2,2-methylenebis(4,6-di-t-butylphenyl) octylphosphite, bis(2,6-di-t-butylphenyl) pentaerythritol-di-phosphite, and tetrakis(2,4-di-t-butylphenyl) 4,4'-biphenylene-di-phosphonite.

From the viewpoints of achieving a high melting point and improving the heat resistance of the resin particles, the antioxidant is preferably at least one selected from the group consisting of dibutylhydroxytoluene, butylhydroxyanisole, propyl gallate, tocopherol, ascorbic acid, catechin chloroformate, a hindered phenol compound, and a phosphate ester compound.

These antioxidants may be used alone or in combination of two or more thereof. The content of the antioxidant is preferably 5 wt% or less, more preferably 0.05 wt% or more and 2 wt% or less, with respect to the total amount of the resin particles.

### (First Layer and Second Layer)

It is preferable that the resin particle according to the present exemplary embodiment successively has a first layer containing at least one cationic resin of a polyalkyleneimine, a polyallylamine, or a polyvinylamine on the surface thereof, and a second layer containing an anionic or nonionic hydrophobic compound.

It is presumed that when the first layer and the second layer are included, it is easier to form the above-mentioned "(4) state where water does not easily penetrate from the surface of the resin particles, but bacteria may enter the resin particles". Therefore, the heat resistance are more excellent.

Hereinafter, the "base particles" refers to target particles on which the first layer and the second layer are formed. Preferably, the target particles on which the first layer and the second layer are formed are particles having the above composition.

### -First Layer-

The first layer is a resin layer on the surface of the base particle. The first layer contains at least one cationic resin of a polyalkyleneimine, a polyallylamine, or a polyvinylamine.

The cationic resin may be any of a polyalkyleneimine, a polyallylamine, and a polyvinyl amine, but a polyalkyleneimine is preferred from the viewpoints of improving the biodegradation rate over time and slowing down the initial biodegradation rate.

From the viewpoints of improving the biodegradation rate over time and slowing down the initial biodegradation rate, the polyalkyleneimine is preferably a polyalkyleneimine having a constituent unit having an alkylene group having 1 or more and 6 or less carbon atoms (preferably 1 or more and 4 or less carbon atoms, and more preferably 1 or more and 2 or less carbon atoms), and more preferably a polyethyleneimine.

In particular, the polyethyleneimine is a compound having high adhesion and high water absorbency. This is because the amino group of the polyethyleneimine has a hydrogen bond with a hydroxy group, an ionic bond with a carboxyl group, and a covalent bond with a carbonyl group. This is because the polyethyleneimine has a polar group (amino group) and a hydrophobic group (ethylene group) in the structure thereof, and therefore has a property of easily binding different substances.

In addition, the polyethyleneimine is a compound having high cationicity. Accordingly, the polyethyleneimine is present as a polycation in water and neutralizes and adsorbs anionic substances.

Further, the polyethyleneimine is a compound having high reactivity because of having a highly reactive primary amino group or secondary amino group. Accordingly, the polyethyleneimine easily reacts with various compounds.

Therefore, when the polyethyleneimine is applied to the polyalkyleneimine, the base particles are more firmly coated with the second layer containing a hydrophobic compound, and the initial biodegradation rate tends to be slowed down while the biodegradation rate over time is obtained.

The number average molecular weight of the cationic resin is preferably 300 or more and 100,000 or less, more preferably 10,000 or more and 85,000 or less, and still more preferably 50,000 or more and 80,000 or less, from the viewpoints of improving the biodegradation rate over time and slowing down the initial biodegradation rate.

The number average molecular weight of the cationic resin is measured by gel permeation chromatography (GPC apparatus: HLC-8320GPC manufactured by Tosoh Corporation, column: TSKgelα-M) in terms of polystyrene using tetrahydrofuran.

### -Second Layer-

The second layer is a compound layer on the first layer. The second layer contains an anionic or nonionic hydrophobic compound.

Examples of the anionic or nonionic hydrophobic compound include a hydrophobic compound having an anionic group (-COOH (carboxyl group), -SO₃H (sulfon group), etc.), and a hydrophobic compound having no cationic or anionic group.

The hydrophobic compound refers to a compound that imparts hydrophobicity (specifically, a water contact angle) to the biodegradable resin particles described later.

Examples of the hydrophobic compound include a silicone compound, a hydrocarbon compound, a fatty acid compound, an acrylic resin, a polyester resin, and a urethane resin.

Among these, at least one selected from the group consisting of a silicone compound, a hydrocarbon compound, a fatty acid compound, an acrylic resin, a polyester resin, and a urethane resin is preferred from the viewpoints of improving the biodegradation rate over time and slowing down the initial biodegradation rate.

Examples of the silicone compound include dimethylpolysiloxane, methylpolysiloxane, octamethylcyclotetrasiloxane, decamethylcyclopentanesiloxane, methylcyclopolysiloxane, various modified silicone oils (an alkyl-modified silicone oil, a polyether-modified silicone oil, an alcohol-modified silicone oil, a fluorine-modified silicone oil, an amino-modified silicone oil, etc.), MQ resin, and silicone rubber.

Among these, the silicone compound is preferably at least one selected from the group consisting of dimethylpolysiloxane, methylpolysiloxane, MQ resin, and silicone rubber from the viewpoints of improving the biodegradation rate over time and slowing down the initial biodegradation rate.

Here, the MQ resin refers to a silicone resin having an M unit which is a monofunctional siloxane unit [(CH₃)₃SiO_{1/2}] and a Q unit which is a tetrafunctional siloxane unit [SiO_{4/2}].

Examples of the commercially available product of the silicone compound include silicone compounds (KM-902, KM-903, KM-910, KM-9729, POLON-MN-ST, KM-9737A, KM-9782, KM-9738A, KM-752T, POLON-MF-33, KM-9717, X-51-1302M (MQ resin), POLON-MF-56, KM-2002-L-1, KM-2002-T, KM-9772, KM-9749, POLON-MF-40, KM-9729, X-52-1133, etc.) manufactured by Shin-Etsu Chemical Co., Ltd., and silicone compounds (BELSII, DM3112VP) manufactured by Wacker Asahikasei Silicone Co., Ltd.

Examples of the hydrocarbon compound include petroleum wax (paraffin wax, microcrystalline wax, petrolatum wax, etc.), and synthetic hydrocarbon wax (polyethylene wax, polypropylene wax, polybutene wax, fischer-tropsch wax, etc.).

Among these, the hydrocarbon compound is preferably at least one selected from the group consisting of paraffin wax, microcrystalline wax, polyethylene wax, and polypropylene wax, from the viewpoints of improving the biodegradation rate over time and slowing down the initial biodegradation rate.

Examples of the commercially available product of the hydrocarbon compound include micro-crystallin (EMUSTAR-0001, etc.) manufactured by Nippon Seiro Co., Ltd., paraffin wax (EMUSTAR-0135, etc.) manufactured by Nippon Seiro Co., Ltd., paraffin wax (AQUACER497 etc.) manufactured by BYK, polyethylene wax (AQUACER507, AQUACER840, AQUACER1547, AQUACER272, etc.) manufactured by BYK Co., Ltd., polyethylene wax (Hi-Tech E-2213, Hi-Tech E-6324, etc.) manufactured by TOHO Chemical Industry Co., Ltd., polypropylene wax (AQUACER593, etc.) manufactured by BYK, and polypropylene (Hi-Tech P-9018, Hi-Tech P-5060P, etc.) manufactured by TOHO Chemical Industry Co., Ltd.

Examples of the fatty acid compound include vegetable oil containing fatty acids (castor oil, tung oil, flaxseed oil, shortening, corn oil, soybean oil, sesame oil, rapeseed oil, sunflower oil, rice bran oil, camellia oil, palm oil, palm oil, walnut oil, olive oil, peanut oil, almond oil, jojoba oil, cacao butter, shea butter, neem oil, safflower oil, japan wax, candelilla wax, rice wax, carnauba wax, etc.).

Among these, at least one selected from the group consisting of carnauba wax, rice wax, candelilla wax, palm wax, castor oil wax, soybean oil wax, and sunflower oil wax is preferred from the viewpoints of improving the biodegradation rate over time and slowing down the initial biodegradation rate.

Examples of the commercially available product of the fatty acid compound include carnauba wax (EMUSTAR-0413 (carnauba wax)) manufactured by Nippon Seiro Co., Ltd., rice wax (AQUASPROUT-7300, etc.) manufactured by Nippon Seiro Co., Ltd., palm wax (AQUASPROUT-7100, etc.) manufactured by Nippon Seiro Co., Ltd., castor oil wax (AQUASPROUT-7500, etc.) manufactured by Nippon Seiro Co., Ltd., soybean oil wax (AQUASPROUT-7200, etc.) manufactured by Nippon Seiro Co., Ltd., sunflower oil wax (AQUASPROUT-7400, etc.) manufactured by Nippon Seiro Co., Ltd., and palm oil wax (Cuckoo Ace TKE, etc.) manufactured by Nippon Seiro Co., Ltd.

Examples of the acrylic resin include well-known acrylic resins such as a polymer of acrylic acid and a polymer of an acrylic acid alkyl ester.

Examples of the commercially available product of the acrylic resin include acrylic resins (3WX-2015, 3MF-320, 3MF-333, 3MF-407, etc.) manufactured by TAISEI FINE CHEMICAL CO., LTD., and acrylic resins (COAT SFC-6440, VONCOAT CE-6270, VONCOAT CE-6400, VONCOAT CF-2800, etc.) manufactured by DIC Corporation.

Examples of the polyester resin include well-known polyester resins such as a polycondensate of a polyhydric carboxylic acid and a polyhydric alcohol and a ring-opening polycondensate of cyclic lactam.

Examples of the commercially available product of the polyester resin include polyester resins (A-110F, A-160P, A-520, A-613D, A-615GE, A-640, A-645GH, A-647GEX, etc.) manufactured by Takamatsu Oil & Fat Co., Ltd.

Examples of the urethane resin include well-known urethane resins such as a polyester-based polyurethane, a polyether-based polyurethane, and a polycarbonate-based polyurethane. Further, as the urethane resin, a material having a urethane polymer shell layer around the acrylic polymer core may be used.

Examples of the commercially available product of the urethane resin include urethane resins (WEM-031U, WEM-200U, WEM-321U, WEM-3000, WBR-016U, WBR-2101, etc.) by TAISEI FINE CHEMICAL CO., LTD.

### -Content of Each Layer-

In the biodegradable resin particle according to the present exemplary embodiment, the mass ratio of the coating amount of the cationic resin in the first layer to the coating amount of the hydrophobic compound in the second layer (coating amount of cationic resin/coating amount of hydrophobic compound) is preferably 0.05 or more and 20 or less, more preferably 0.1 or more and 10 or less, and still more preferably 0.1 or more and 3 or less, from the viewpoints of improving the biodegradation rate over time and slowing down the initial biodegradation rate.

In addition, the content of the cationic resin with respect to the base particles is preferably 0.05 mass% or more and 15 mass% or less, more preferably 0.1 mass% or more and 10 mass% or less, and still more preferably 0.1 mass% or more and 3 mass% or less, from the viewpoints of improving the biodegradation rate over time and slowing down the initial biodegradation rate.

Further, the content of the hydrophobic compound with respect to the base particles is preferably 0.05 mass% or more and 15 mass% or less, more preferably 0.1 mass% or more and 10 mass% or less, and still more preferably 0.1 mass% or more and 3 mass% or less, from the viewpoints of improving the biodegradation rate over time and slowing down the initial biodegradation rate.

Here, each coating amount of the cationic resin and the hydrophobic compound (that is, each coating amount of the first layer and the second layer) is measured as follows. The coating amount of the cationic resin is determined by the difference between the treated amount of the cationic resin and the cationic resin obtained by drying the supernatant after the treatment. Similarly, the coating amount of the hydrophobic compound is determined by the difference between the treated amount of the hydrophobic compound and the hydrophobic compound obtained by drying the supernatant after the treatment.

### (Melting Point)

The resin particle according to the present exemplary embodiment has a melting point of 170°C or higher.

The melting point is a value measured using a melting point measurement device.

As the melting point measurement device, for example, "MP80" (manufactured by METTLER TOLEDO) is used. The melting point is measured by heating 5 mg of a sample from 20°C to 300°C at a constant heating rate (5°C/min).

From the viewpoint of further enhancing the heat resistance of the resin particles, the melting point is preferably 190°C or higher and 290°C or lower, more preferably 200°C or higher and 270°C or lower, and still more preferably 210°C or higher and 250°C or lower.

### (Vickers Hardness)

The resin particle according to the second exemplary embodiment has a Vickers hardness of 900 HV or less.

The Vickers hardness is measured by the method described in JIS Z2244: 2009.

The specific measurement conditions are as follows.

The temperature during the measurement is set to 25°C.

One obtained by leveling 1 g of a sample and fixing the same on a slide glass with an epoxy adhesive is used as a test piece, and an indenter is pushed from above the sample to measure the Vickers hardness.

The test force is set to 294.2 N (= 30 kgf), and the holding time of the test force is set to 20 seconds.

From the viewpoint of further enhancing the flexibility of the resin particles, the Vickers hardness is preferably 200 HV or more and 800 HV or less, more preferably 250 HV or more and 700 HV or less, and still more preferably 300 HV or more and 600 HV or less.

### (Volume Average Particle Diameter and Large-diameter Side Particle Size Distribution Index GSDv)

From the viewpoint of further enhancing the heat resistance and the flexibility of the resin particles, the resin particle according to the present exemplary embodiment preferably has a volume average particle diameter of 3 µm or more and 100 µm or less, and a large-diameter side particle size distribution index GSDv of 1.50 or less.

The volume average particle diameter is more preferably 5 µm or more and 70 µm or less, and still more preferably 8 µm or more and 60 µm or less.

The large-diameter side particle size distribution index GSDv is more preferably 1.3 or less, and still more preferably 1.2 or less.

The volume average particle diameter and the large-diameter side particle size distribution index GSDv of the resin particle are measured as follows.

The particle diameter is measured by the LS particle size distribution measurement device "Beckman Coulter LS13 320 (manufactured by Beckman Coulter Inc.)", the cumulative distribution of the particle diameter is drawn from the small diameter side on a volume basis, and the particle diameter at a cumulative 50% is obtained as the volume average particle diameter.

On the other hand, the cumulative distribution of particle diameter is drawn from the small diameter side on a volume basis, and the particle diameter at a cumulative total of 50% is defined as the number average particle diameter D50v, and the particle diameter at a cumulative 84% is defined as the number particle diameter D84v. Then, the large-diameter side number particle size distribution index GSDv is calculated by the formula GSDv = (D84v/D50v)^{1/2}.

### (Combination of Composition)

The resin particles according to the present exemplary embodiment are preferably, but are not limited to, resin particles having the following composition.

### · Resin particle example (1)

Resin particle containing:
at least one selected from the group consisting of a cellulose acylate (A), a polyamide compound (B) and an aliphatic polyester compound (C); and
at least one selected from the group consisting of an aromatic compound (D) containing a phenolic hydroxy group and a hydrocarbon group having 8 or more and 20 or less carbon atoms and an ester compound (E),
in which the ester compound (E) is contained in an amount of 0 mass% or more and less than 30 mass% with respect to a total amount of the resin particles.

### · Resin particle example (2)

Resin particle containing:
a first layer and a second layer which are provided in this order at a surface of a base particle so that the first layer is provided between the base particle and the second layer,
in which the base particle contains a cellulose acylate (A); and an aromatic compound (D) having a long-chain aliphatic group having 8 or more and 20 or less carbon atoms and having at least one of a phenolic hydroxy group or a monoglycidyl ether group directly bonded to the aromatic group,
in which the first layer contains at least one of a polyalkyleneimine, a polyallylamine, or a polyvinylamine as a cationic resin, and
in which the second layer contains an anionic or nonionic hydrophobic compound.

### · Resin particle example (3)

Resin particle containing:
a cellulose acylate (A); and
an ester compound (E),
in which the ester compound (E) is contained in an amount of less than 30 mass% with respect to a total amount of the resin particles.

### · Resin particle example (4)

Resin particle containing:
a first layer and a second layer which are provided in this order at a surface of a base particle so that the first layer is provided between the base particle and the second layer,
in which the base particle contains a cellulose acylate (A); and an ester compound (E), in which the ester compound (E) is contained in an amount of less than 30 mass% with respect to a total amount of the resin particles,
in which the first layer contains at least one of a polyalkyleneimine, a polyallylamine, or a polyvinylamine as a cationic resin, and
in which the second layer contains an anionic or nonionic hydrophobic compound.

### · Resin particle example (5)

Resin particle containing:
a first layer and a second layer which are provided in this order at a surface of a base particle so that the first layer is provided between the base particle and the second layer,
in which the base particle contains a polyamide compound (B); and an aromatic compound (D) having a long-chain aliphatic group having 8 or more and 20 or less carbon atoms and having at least one of a phenolic hydroxy group or a monoglycidyl ether group directly bonded to the aromatic group,
in which the first layer contains at least one of a polyalkyleneimine, a polyallylamine, or a polyvinylamine as a cationic resin, and
in which the second layer contains an anionic or nonionic hydrophobic compound.

### · Resin particle example (6)

Resin particle containing:
a first layer and a second layer which are provided in this order at a surface of a base particle so that the first layer is provided between the base particle and the second layer,
in which the base particle contains a polyamide compound (B); and an ester compound (E),
in which the first layer contains at least one of a polyalkyleneimine, a polyallylamine, or a polyvinylamine as a cationic resin, and
in which the second layer contains an anionic or nonionic hydrophobic compound.

### · Resin particle example (7)

Resin particle containing:
a first layer and a second layer which are provided in this order at a surface of a base particle so that the first layer is provided between the base particle and the second layer,
in which the base particle contains an aliphatic polyester compound (C),
in which the first layer contains at least one of a polyalkyleneimine, a polyallylamine, or a polyvinylamine as a cationic resin, and
in which the second layer contains an anionic or nonionic hydrophobic compound.

### · Resin particle example (8)

Resin particle containing:
a first layer and a second layer which are provided in this order at a surface of a base particle so that the first layer is provided between the base particle and the second layer,
in which the base particle contains an aliphatic polyester compound (C); and an aromatic compound (D) having a long-chain aliphatic group having 8 or more and 20 or less carbon atoms and having at least one of a phenolic hydroxy group or a monoglycidyl ether group directly bonded to the aromatic group,
in which the first layer contains at least one of a polyalkyleneimine, a polyallylamine, or a polyvinylamine as a cationic resin, and
in which the second layer contains an anionic or nonionic hydrophobic compound.

### · Resin particle example (9)

Resin particle containing:
a first layer and a second layer which are provided in this order at a surface of a base particle so that the first layer is provided between the base particle and the second layer,
in which the base particle contains an aliphatic polyester compound (C); and an ester compound (E),
in which the first layer contains at least one of a polyalkyleneimine, a polyallylamine, or a polyvinylamine as a cationic resin, and
in which the second layer contains an anionic or nonionic hydrophobic compound.

### <Method for Producing Resin Particles>

Examples of the method for producing the resin particles include the following methods.
(1) a kneading and crushing method in which each component is kneaded and the obtained kneaded product is crushed and classified to obtain granules
(2) a dry process method in which the granules obtained by the kneading and crushing method are changed in shape by mechanical impact force or thermal energy to obtain granules
(3) an agglomeration and coalescence method in which particle dispersions of respective components are mixed, and particles in the dispersion are agglomerated and heat-fused to obtain granules
(4) a dissolution and suspension method in which an organic solvent in which each component is dissolved is suspended in an aqueous solvent to granulate granules containing each component
(5) a kneading and dissolving method in which each component and a binder are kneaded and extruded to form pellets, and the obtained pellets are stirred in a solvent that dissolves only the binder obtain granules

Examples of the method for producing the resin particles including the first layer and the second layer include the following methods.

### -First Step-

In the first step, the base particles are prepared.

Examples of the method for producing the base particles include the methods (1) to (5) for producing the resin particles.

Next, an aqueous dispersion in which the obtained base particles are dispersed is prepared. It is preferable to acid-wash the base particles before preparing the aqueous dispersion.

Next, the aqueous dispersion, the aqueous dispersion in which the base particles are dispersed, and an aqueous solution containing a cationic resin are mixed. Thereby, for example, the hydroxy group of the resin contained in the base particles reacts with the amine site of the cationic resin to form the first layer.

### -Second Step-

In the second step, the base particles on which the first layer is formed are taken out from the mixed solution. Taking out the base particles is carried out, for example, by filtering the mixed solution. The base particles taken out may be washed with water. Thereby, the unreacted cationic resin can be removed.

Next, an aqueous dispersion in which the base particles are dispersed is prepared, and then the aqueous dispersion and an emulsion solution of an anionic or nonionic hydrophobic compound are mixed. Thereby, the emulsion of the hydrophobic compound is adsorbed on the first layer of the base particles.

Thereafter, when the mixed solution is dried, the emulsion of the hydrophobic compound is broken, and the hydrophobic compound is filmed on the first layer. Thereby, the second layer is formed.

With the above steps, the resin particles according to the present exemplary embodiment can be obtained.

### <Uses>

Examples of the uses of the resin particles according to the present exemplary embodiment include granules of cosmetic base materials, rolling agents, abrasives, scrubbing agents, display spacers, bead molding materials, light diffusing particles, resin strengthening agents, refractive index control agents, biodegradation accelerators, fertilizers, water-absorbent particles, toner particles, and anti-blocking particles.

Among these, a cosmetic base material is preferable for the use of the resin particles according to the present exemplary embodiment.

Since the resin particles according to the present exemplary embodiment are excellent in heat resistance, when used as a cosmetic base material, the resin particles are less likely to be deformed or denatured by heat even when the cosmetic is coated onto the skin for a long time or when the cosmetic is placed in a high temperature state during production.

In addition, since the resin particles according to the present exemplary embodiment are excellent in flexibility, when used as a cosmetic base material, the cosmetic spreads well on the skin, and the part onto which the cosmetic is coated tends to have a good skin touch feeling.

Specific examples of the cosmetic base material include cosmetic base materials such as base makeup cosmetics (makeup base, concealer, foundation, face powder, etc.), makeup cosmetics (lipstick, gloss, lip liner, teak, eyeshadow, eyeliner, mascara, eyebrow, nails, nail care cosmetics, etc.), and skin care cosmetics (facial cleansers, cleansers, lotions, emulsions, serums, facial masks, eye and mouth care cosmetics, etc.).

In particular, the resin particles according to the present exemplary embodiment is preferably used as a cosmetic base material for makeup cosmetics from the viewpoint that the cosmetic base material for makeup cosmetics is required to have flexibility, heat resistance, and biodegradability.

Here, the cosmetic base material means a component for holding a dosage form of a cosmetic composition.

### Examples

Examples will be described below, but the present invention is not limited to these Examples. In the following description, all "parts" and "%" are based on mass unless otherwise specified. Examples 28, 29, 33, and 52 are of the invention as claimed.

### <Preparation of Each Material>

The materials shown in Tables 1 and 2 are prepared.
"Number of type of substituent" in Table 1 indicates the number of types of acyl groups contained in the cellulose acylate (A).
"Number of carbon atoms of constituent unit" in Table 1 indicates the number of the atoms of the constituent unit of the polyamide compound (B).
"Molecular weight" in Table 1 indicates the molecular weight of the ester compound (E).

**[Table 1]**

| Category | No. | Compound name | Product name | Manufacturer | Number of type of substituent | Number of carbon atoms of constituent unit | Molecular weight |
|---|---|---|---|---|---|---|---|
| Cellulose acylate (A) | CA1 | Cellulose acetate butyrate | CAB381-20 | Eastman Chemical | 2 | | |
| | CA2 | Cellulose acetate butyrate | CAB171-1.5 | Eastman Chemical | 2 | | |
| | CA3 | Cellulose acetate propionate | CAP482-20 | Eastman Chemical | 2 | | |
| | CA4 | Diacetyl cellulose | L50 | Daicel | 1 | | |
| Polyamide compound (B) | PA1 | Polyamide 12 | DAIAMID | Daicel Evonik | | 12 | |
| | PA2 | Polyamide 11 | Rilsan | Arkema | | 11 | |
| | PA3 | Polyamide 16 | AMIRAN CM1017 | TORAY | | 6 | |
| | PA4 | Polyamide 610 | AMIRAN CM2001 | TORAY | | 16 | |
| Aliphatic polyester compound (C) | PE1 | Polyhydroxy alkanoate | Aonilex 151X | KANEKA | | | |
| | PE2 | Polylactic acid | Ingeo3001D | NatureWorks | | | |
| | PE3 | Polybutylene succinate | BioPBS | Mitsubishi Chemical | | | |
| Aromatic compound (D) | PH1 | Cardanol | NX2026 | Cardlite | | | |
| | PH2 | Epoxy-modified cardanol | Ultra LITE 513 | Cardlite | | | |
| | PH3 | Phenol novolac type epoxy resin | EPICLON865-alkyl-modified product | DIC | | | |
| Ester compound (E) | ES1 | Diisopropyl adipate | NIKKOL DID | NIKKOL | | | 230 |
| | ES2 | Diisobutyl adipate | Vinisizer-40 | KAO | | | 258 |
| | ES3 | Triethyl citrate | Citroflex 2 | MORIMURA BROS | | | 276 |
| | ES4 | Acetyl tributyl citrate | Citroflex A4 | MORIMURA BROS | | | 402 |
| | ESS | Isopropyl sebacate | NIKKOL DIS | NIKKOL | | | 286 |
| | ES6 | Isooctadecyl adipate | Isooctadecyl adipate | FUJI FILM Wako Pure | | | 651 |
| | ES7 | Dimethyl adipate | Dimethyl adipate | FUJI FILM Wako Pure | | | 174 |
| Oxidative biodegradable resin | OB-1 | Polyethylene/P-life SMC2360 20% | P-LIFE GREEN20 | P-Life Japan | | | |
| | OB-2 | Polypropylene P-life SMC2360 20% | P-LIFE PP20 | P-Life Japan | | | |
| Other resin | OR-1 | Polymethyl methacrylate | DELPET 720V | Asahi Kasei | | | |

**[Table 2]**

| Category | No. | Compound name | Product name | Manufacturer |
|---|---|---|---|---|
| Others | AD1 | Dibutylhydroxytoluene | Dibutylhydroxytoluene | FUJI FILM Wako Pure |
| | AD2 | Butylated hydroxyanisole | Butylated hydroxyanisole | FUJI FILM Wako Pure |
| | AD3 | Propyl gallate | Propyl gallate | Fuji Chemical Industries |
| | AD4 | Tocopherol | Tocopherol | Mitsubishi Chemical Food |
| | AD5 | Ascorbic acid | Vitamin C | FUSO CHEMICAL |
| | AD6 | Chloroformate catechin | Chloroformate catechin | FUJI FILM Wako Pure |
| | AD7 | Pentaerythrityl tetrakis(3-(3,5-di-t-butyl-4-hydroxyphenyl) propionate) | Irganox 1010 | BASF |
| | AD8 | Tris(2,4-di-t-butylphenyl) phosphite | Irgafos 168 | BASF |
| | AD9 | Tea extract | Sunfood 100 | Mitsubishi Chemical |
| First layer | 1S-1 | Polyethyleneimine | EPOMIN P-1000 | NIPPON SHOKUBAI |
| | 1S-2 | Polyethyleneimine | EPOMIN SP-006 | NIPPON SHOKUBAI |
| | 1S-3 | Polyallylamine | PAA-MCL-3L | NITTOBO MEDICAL |
| | 1S-4 | Polyvinylamine | PVAM | Mitsubishi Chemical |
| Second layer | 2S-1 | Dimethylpolysiloxane | KM-2729 | Shin-Etsu Chemical |
| | 2S-2 | Paraffin wax | EMUST-AR0135 | Nippon Seiro |
| | 2S-3 | Carnauba wax | EMUST-AR0413 | Nippon Seiro |

### <Examples 1 to 57 and Comparative Examples 1 to 11>

The procedures of each Example and each Comparative Example are described below.

### (Preparation of Resin Pellet)

### -Examples 1 to 57 and Comparative Examples 1 to 11-

The cylinder temperature is adjusted according to the charged composition ratios shown in Table 3, and kneading is carried out with a twin-screw kneader (TEX41SS manufactured by Toshiba Machine Co., Ltd.) to obtain a pellet-shaped resin composition (hereinafter referred to as a resin pellet).

### (Preparation of Base Particles -Solvent System-)

### -Examples 1 to 27, 49, 50, 55 to 59, 62 to 65 and Comparative Examples 1 to 4-

300 g of the resin pellet is completely dissolved in 700 g of methyl ethyl ketone. The mixture is added to an aqueous liquid in which 100 g of calcium carbonate, 4 g of carboxymethyl cellulose and 200 g of methyl ethyl ketone are dispersed in 1100 g of pure water, and the mixture is stirred for 3 hours (hereinafter, the stirring is referred to as "aqueous liquid stirring"). 10 g of sodium hydroxide is added thereto, and the mixture is heated to 80°C and stirred for 3 hours to remove methyl ethyl ketone. The residue is filtered to obtain the base particles.

### -Examples 44 to 47-

Base particles are obtained in the same manner as in Example 1 except that the stirring time of the aqueous liquid is changed to 5 hours, 1 hour, 8 hours, and 30 minutes.

### -Example 48-

Base particles are obtained in the same manner as in Example 1 except that the amount of calcium carbonate added is changed to 50 g.

### (Preparation of Base Particles -Kneading System-)

### -Examples 28 to 43, 51 to 54, 60, 61 and Comparative Examples 5 to 9-

300 g of resin pellets and 700 g of polyacrylic acid (Julimer AC-10P manufactured by TOAGOSEI CO., LTD.) are premixed and kneaded with a twin-screw kneader (TEX41SS manufactured by Toshiba Machine Co., Ltd.) at a cylinder temperature of 220°C to 240°C to obtain a pellet-shaped resin composition. The resin composition is stirred in distilled water with a mass of 10 times for 1 hour to dissolve polyacrylic acid, and the residue is filtered to obtain the base particles.

### (Preparation of Resin Particles Free of First Layer and Second Layer)

### -Examples 1 to 10, 42, 43, 49 to 56 and Comparative Examples 1 to 3, 5 to 9-

The base particles obtained in each example are freeze-dried to obtain resin particles.

### -Comparative Example 10-

Commercially available cellulose particles (CELLULOBEADS D5, manufactured by DAITO KASEI KOGYO CO., LTD.) are used as the resin particles.

### -Comparative Example 11-

Commercially available cellulose particles (CELLULOBEADS D10, manufactured by DAITO KASEI KOGYO CO., LTD.) are used as the resin particles.

### (Preparation of Resin Particles Including First Layer and Second Layer)

### -Examples 11 to 41, 44 to 48, 57 to 65 and Comparative Example 4-

The base particles obtained in each example are dispersed in pure water to prepare a slurry having a solid content of 20 mass%. The cationic resin solution in an amount shown in Table 3 is added with respect to the amount of the solid content contained in this slurry in terms of pure content, and stirring is performed under the condition of 25°C for 1 hour. After the stirring is completed, the residue is filtered and the obtained substance is dispersed in pure water again to adjust the solid content to 20%, a hydrophobic compound in an amount shown in Table 3 is added with respect to the amount of the solid content contained in this slurry in terms of pure content, and stirring is performed under the condition of 25°C for 1 hour. After the stirring is completed, the residue is filtered and the solid content is freeze-dried to obtain resin particles.

### <Various Measurements>

According to the methods described above, the volume average particle diameter, the large-diameter side particle size distribution index GSDv, the mass loss rate in distilled water, the mass loss rate in compost, the melting point, and the Vickers hardness of the resin particles are measured. As the compost, a compost bacteria bed manufactured by IRIS OHYAMA Inc. is used.

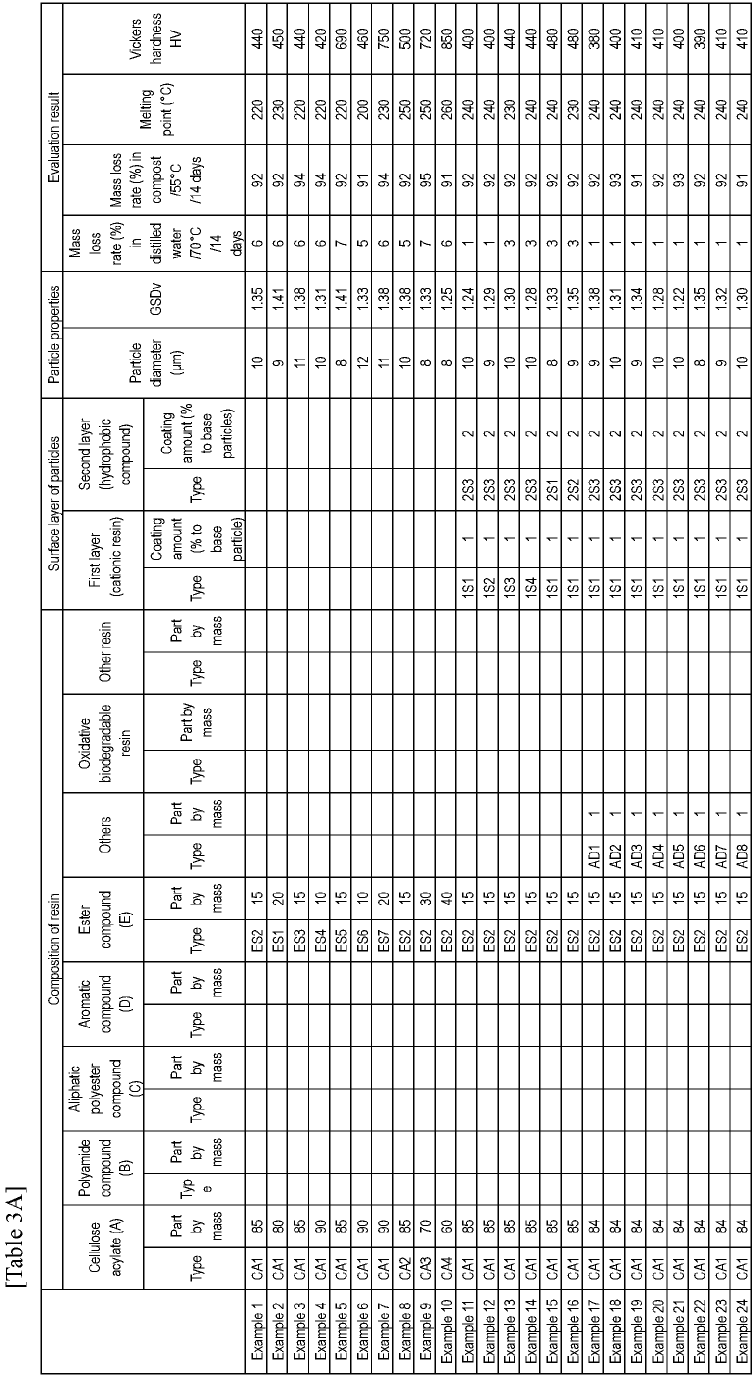

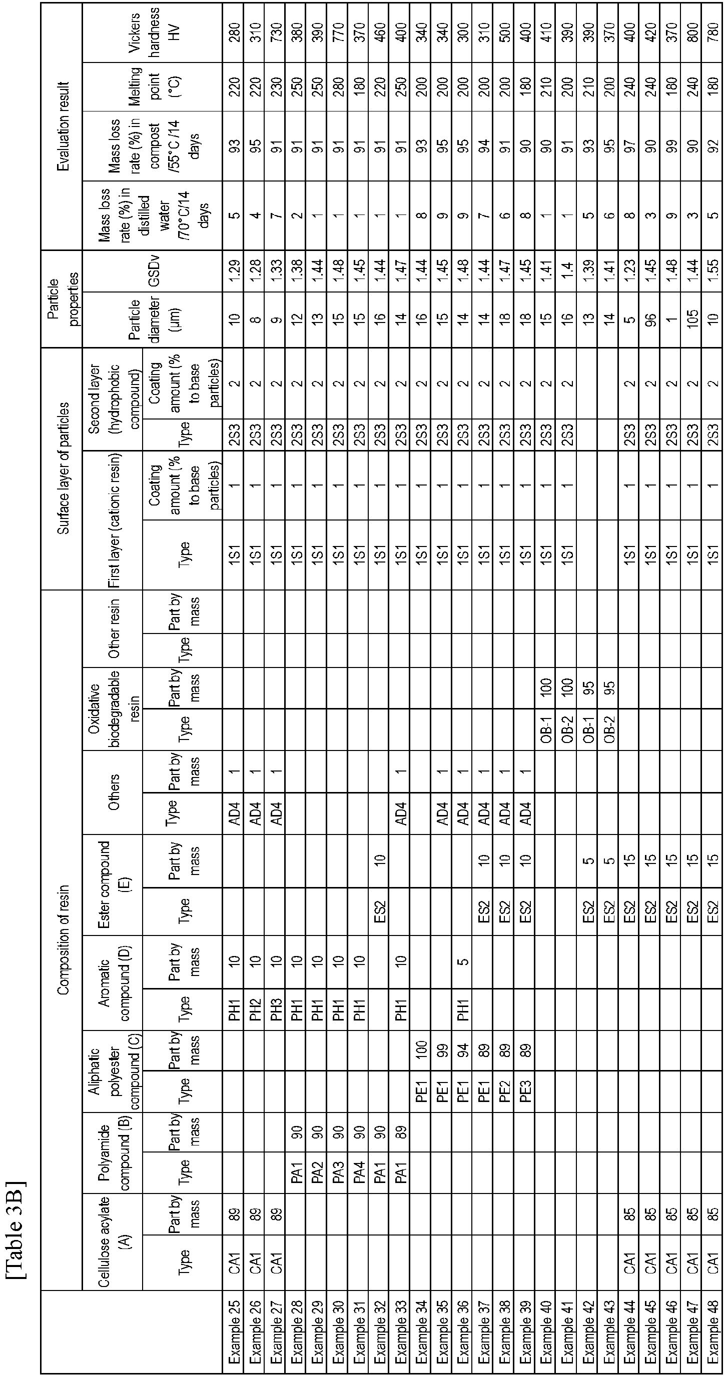

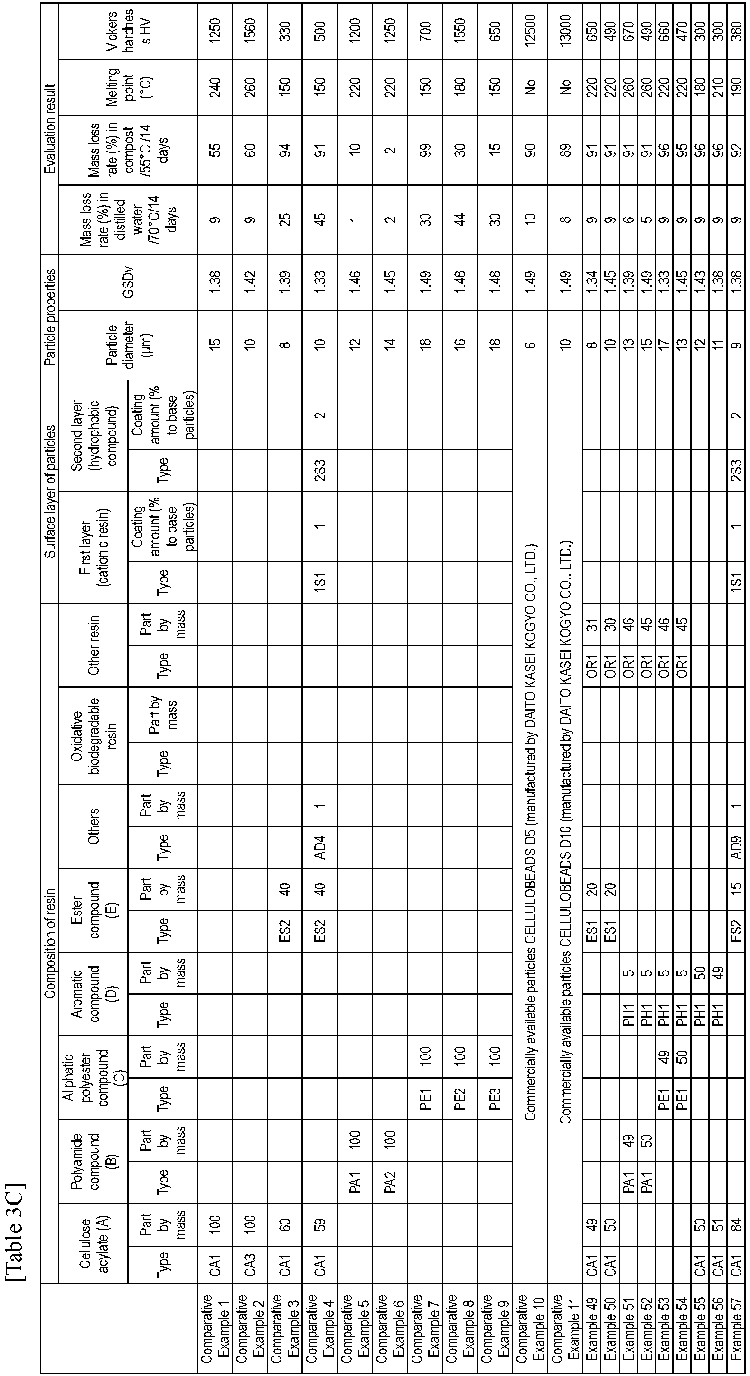

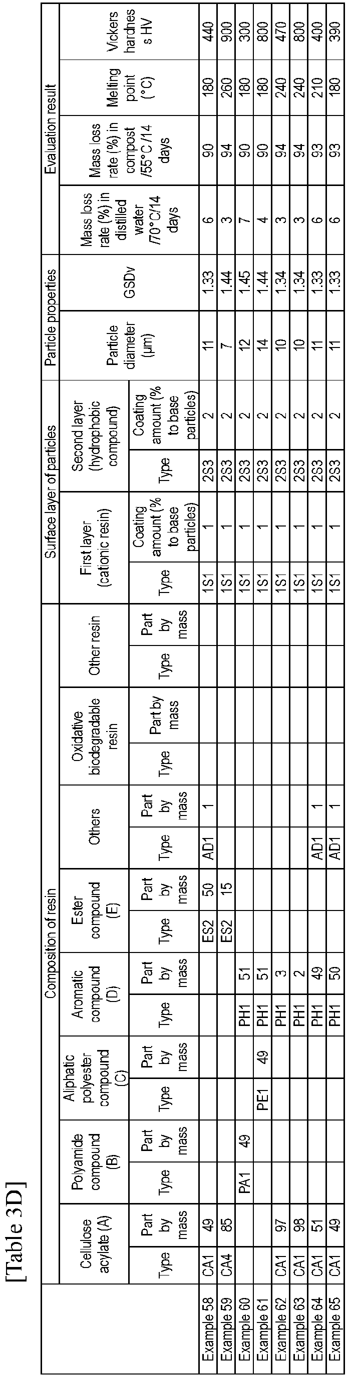

From the above results, it can be seen that the resin particles of Examples are excellent in heat resistance and flexibility.

It can be also seen that because of being excellent in heat resistance and flexibility, the resin particles of Examples are suitable as a cosmetic base material.

The foregoing description of the exemplary embodiments of the present invention has been provided for the purposes of illustration and description. It is not intended to be exhaustive or to limit the invention to the precise forms disclosed. Obviously, many modifications and variations will be apparent to practitioners skilled in the art. The embodiments were chosen and described in order to best explain the principles of the invention and its practical applications, thereby enabling others skilled in the art to understand the invention for various embodiments and with the various modifications as are suited to the particular use contemplated.

## Claims

1. A resin particle,
wherein a mass loss rate after being immersed in distilled water at 70°C for 14 days is less than 10%, and
wherein a mass loss rate after being left in a compost at 55°C for 14 days is 90% or more, the compost having nitrogen of 1.3 mass%, phosphoric acid of 0.5 mass%, and potassium of 2.9 mass% with respect to a total amount of the compost, and the compost having a carbon-nitrogen ratio (C/N ratio) of 25,
comprising:
a polyamide compound (B1) having a constituent unit having 8 or more and 15 or less carbon atoms, said compound (B1) being present
in an amount of 50 mass% or more with respect to a total amount of the resin particle; and
an aromatic compound (D) in an amount of 3 mass% or more and less than 50 mass% with respect to the total amount of the resin particle,
wherein the aromatic compound (D) has a long-chain aliphatic group having 8 or more and 20 or less carbon atoms and at least one of a phenolic hydroxy group or a monoglycidyl ether group that directly bonds to an aromatic group of the aromatic compound (D).

2. The resin particle according to claim 1,
wherein the aromatic compound (D) is a cardanol compound (D1).

3. The resin particle according to any one of claims 1 to 2, comprising:
an ester compound (E1) having a molecular weight of 200 or more and less than 500 in an amount of 5 mass% or more and less than 30 mass% with respect to the total amount of the resin particle.

4. The resin particle according to claim 3,
wherein the ester compound (E1) is at least one of an adipate ester or a citrate ester.

5. The resin particle according to any one of claims 1 to 4, comprising:
at least one selected from the group consisting of dibutylhydroxytoluene, butylhydroxyanisole, propyl gallate, tocopherol, ascorbic acid, catechin chloroformate, a hindered phenol compound, and a phosphate ester compound.

6. The resin particle according to any one of claims 1 to 5,
wherein a first layer and a second layer are provided in this order at a surface of a base particle so that the first layer is provided between the base particle and the second layer,
wherein the first layer comprises at least one of a polyalkyleneimine, a polyallylamine, or a polyvinylamine as a cationic resin, and
wherein the second layer comprises an anionic or nonionic hydrophobic compound.

7. The resin particle according to any one of claims 1 to 6, having a volume average particle diameter of 3 µm or more and 100 µm or less as measured in accordance with the description, and a large-diameter side particle size distribution index GSDv of 1.50 or less as measured in accordance with the description.

8. Use of the resin particle according to any one of claims 1 to 7, as a cosmetic base material.

## Patentansprüche

1. Harzpartikel,
wobei die Masseverlustrate nach 14-tägigem Eintauchen in destilliertes Wasser bei 70 °C weniger als 10 % beträgt und wobei die Masseverlustrate nach 14-tägigem Liegenlassen in einem Kompost bei 55 °C 90 % oder mehr beträgt, der Kompost 1,3 Massen-% Stickstoff, 0,5 Massen-% Phosphorsäure und 2,9 Massen-% Kalium, in Bezug auf die Gesamtmenge des Komposts, enthält und der Kompost ein Kohlenstoff-Stickstoff-Verhältnis (C/N-Verhältnis) von 25 aufweist,
umfassend:
eine Polyamidverbindung (B1) mit einer konstituierenden Einheit mit 8 oder mehr und 15 oder weniger Kohlenstoffatomen, wobei die Verbindung (B1) in einer Menge von 50 Massen-% oder mehr in Bezug auf eine Gesamtmenge des Harzpartikels vorhanden ist; und
eine aromatische Verbindung (D) in einer Menge von 3 Massen-% oder mehr und weniger als 50 Massen-% in Bezug auf die Gesamtmenge des Harzpartikels,
wobei die aromatische Verbindung (D) eine langkettige aliphatische Gruppe mit 8 oder mehr und 20 oder weniger Kohlenstoffatomen und mindestens eine phenolische Hydroxygruppe oder eine Monoglycidylethergruppe aufweist, die direkt an eine aromatische Gruppe der aromatischen Verbindung (D) bindet.

2. Harzpartikel nach Anspruch 1,
wobei die aromatische Verbindung (D) eine Cardanolverbindung (D1) ist.

3. Harzpartikel nach einem der Ansprüche 1 bis 2, umfassend:
eine Esterverbindung (E1) mit einem Molekulargewicht von 200 oder mehr und weniger als 500 in einer Menge von 5 Massen-% oder mehr und weniger als 30 Massen-% in Bezug auf die Gesamtmenge des Harzpartikels.

4. Harzpartikel nach Anspruch 3,
wobei die Esterverbindung (E1) mindestens eines von einem Adipatester oder einem Citratester ist.

5. Harzpartikel nach einem der Ansprüche 1 bis 4, umfassend: mindestens eines ausgewählt aus der Gruppe bestehend aus Dibutylhydroxytoluol, Butylhydroxyanisol, Propylgallat, Tocopherol, Ascorbinsäure, Catechinchlorformiat, einer gehinderten Phenolverbindung und einer Phosphatesterverbindung.

6. Harzpartikel nach einem der Ansprüche 1 bis 5,
wobei eine erste Schicht und eine zweite Schicht in dieser Reihenfolge auf einer Oberfläche eines Basispartikels bereitgestellt sind, so dass die erste Schicht zwischen dem Basispartikel und der zweiten Schicht bereitgestellt ist,
wobei die erste Schicht mindestens eines von einem Polyalkylenimin, einem Polyallylamin oder einem Polyvinylamin als kationisches Harz umfasst und
wobei die zweite Schicht eine anionische oder nichtionische hydrophobe Verbindung umfasst.

7. Harzpartikel nach einem der Ansprüche 1 bis 6, das einen volumengemittelten Partikeldurchmesser von 3 µm oder mehr und 100 µm oder weniger, wie gemäß der Beschreibung gemessen, und einen Partikelgrößenverteilungsindex GSDv auf der Seite des großen Durchmessers von 1,50 oder weniger, wie gemäß der Beschreibung gemessen, aufweist.

8. Verwendung des Harzpartikels nach einem der Ansprüche 1 bis 7 als kosmetisches Grundmaterial.

## Revendications

1. Particule de résine,
dans laquelle un taux de perte de masse après immersion dans de l'eau distillée à 70 °C pendant 14 jours est inférieur à 10 %, et
dans laquelle un taux de perte de masse après avoir été laissé dans un compost à 55 °C pendant 14 jours est de 90 % ou plus, le compost contenant de l'azote à raison de 1,3 % en masse, de l'acide phosphorique à raison de 0,5 % en masse et du potassium à raison de 2,9 % en masse par rapport à une quantité totale du compost, et le compost ayant un rapport carbone-azote (rapport C/N) de 25,
comprenant :
un composé polyamide (B1) ayant un motif constitutif ayant 8 ou plus et 15 ou moins atomes de carbone, ledit composé (B1) étant présent en une quantité de 50 % en masse ou plus par rapport à une quantité totale de la particule de résine ; et
un composé aromatique (D) en une quantité de 3 % en masse ou plus et inférieure à 50 % en masse par rapport à la quantité totale de la particule de résine,
dans laquelle le composé aromatique (D) comprend un groupe aliphatique à longue chaîne ayant 8 ou plus et 20 ou moins atomes de carbone et au moins un groupe parmi un groupe hydroxy phénolique ou un groupe monoglycidyléther qui se lie directement à un groupe aromatique du composé aromatique (D).

2. Particule de résine selon la revendication 1,
dans laquelle le composé aromatique (D) est un composé cardanol (D1) .

3. Particule de résine selon l'une quelconque des revendications 1 et 2, comprenant :
un composé ester (E1) ayant un poids moléculaire de 200 ou plus et inférieur à 500 en une quantité de 5 % en masse ou plus et inférieure à 30 % en masse par rapport à la quantité totale de la particule de résine.

4. Particule de résine selon la revendication 3,
dans laquelle le composé ester (E1) est au moins un ester parmi un ester adipate ou un ester citrate.

5. Particule de résine selon l'une quelconque des revendications 1 à 4, comprenant :
au moins un composé choisi dans le groupe constitué du dibutylhydroxytoluène, du butylhydroxyanisole, du gallate de propyle, du tocophérol, de l'acide ascorbique, du chloroformiate de catéchine, d'un composé phénolique encombré et d'un composé ester phosphate.

6. Particule de résine selon l'une quelconque des revendications 1 à 5,
dans laquelle une première couche et une seconde couche sont disposées dans cet ordre à une surface d'une particule de base de sorte que la première couche est disposée entre la particule de base et la seconde couche,
dans laquelle la première couche comprend au moins l'une parmi une polyalkylèneimine, une polyallylamine ou une polyvinylamine en tant que résine cationique, et
dans laquelle la seconde couche comprend un composé hydrophobe anionique ou non ionique.

7. Particule de résine selon l'une quelconque des revendications 1 à 6, ayant un diamètre moyen de particule en volume de 3 µm ou plus et de 100 µm ou moins tel que mesuré conformément à la description, et un indice de distribution granulométrique latérale de grand diamètre GSDv de 1,50 ou moins, mesuré conformément à la description.

8. Utilisation de la particule de résine selon l'une quelconque des revendications 1 à 7, comme matériau cosmétique de base.
